(19) Europäisches Patentamt
European Patent Office
Office européen des brevets

(11) EP 4 620 997 A1

(12) EUROPEAN PATENT APPLICATION
published in accordance with Art. 153(4) EPC

(43) Date of publication:
24.09.2025 Bulletin 2025/39

(21) Application number: 23891672.0

(22) Date of filing: 17.11.2023

(51) International Patent Classification (IPC):
$C08J\ 3/16$ (2006.01)    $A61K\ 8/73$ (2006.01)
$A61Q\ 1/02$ (2006.01)    $A61Q\ 17/04$ (2006.01)
$A61Q\ 19/00$ (2006.01)    $C08B\ 15/08$ (2006.01)

(52) Cooperative Patent Classification (CPC):
A61K 8/73; A61Q 1/02; A61Q 17/04; A61Q 19/00;
C08B 15/08; C08J 3/16

(86) International application number:
PCT/JP2023/041400

(87) International publication number:
WO 2024/106527 (23.05.2024 Gazette 2024/21)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(30) Priority: 17.11.2022 JP 2022183842

(71) Applicant: Kao Corporation
Chuo-ku,
Tokyo 103-8210 (JP)

(72) Inventors:
• HAYASHI, Yuki
  Wakayama-shi, Wakayama 640-8580 (JP)
• SATO, Kazuaki
  Wakayama-shi, Wakayama 640-8580 (JP)
• ISHIYAMA, Shogo
  Toyohashi-shi, Aichi 441-8074 (JP)

(74) Representative: Hoffmann Eitle
Patent- und Rechtsanwälte PartmbB
Arabellastraße 30
81925 München (DE)

(54) POROUS CELLULOSE PARTICLES

(57) Porous cellulose particles having a compression modulus of 50 MPa or less, a specific surface area of 100 $m^2/g$ or greater and less than 500 $m^2/g$, and a pore volume of 1.5 mL/g or more.

**Description**

Technical Field

The present invention relates to porous cellulose particles.

Background Art

It is known that functional polymer particles are used as additives for cosmetics for the purpose of imparting a soft touch feel (an elastic feel), a smooth touch feel, or a sebum capturing function or the like. Most of these polymer particles contain a synthetic polymer (microplastic beads) having a particle size of several $\mu$m. However, from the viewpoint of environmental concerns, the use of microplastic beads is expected to be regulated in the future.

Therefore, polymer particles made of a biodegradable natural polymer material have attracted attention as particles which are not classified into the microplastic beads. Examples of the polymer particles include cellulose particles without chemical modification.

As functional cellulose particles used in cosmetics, porous cellulose particles are known. The cellulose particles are preferably porous in that a functional substance in a cosmetic can be included inside the particles.

For example, Patent Document 1 discloses porous micro cellulose particles used as an additive for cosmetics or the like, the particles having a crystal form I, a porous structure in which the specific surface area is 20 m$^2$/g or greater, and the volume of pores with a size of 0.01 $\mu$m or greater is 0.3 cm$^3$/g or greater, and an average particle size of at most 100 $\mu$m.

Patent Document 2 discloses porous cellulose particles formed by aggregation of crystalline cellulose of crystal form I, the porous cellulose particles having an average particle size $d_1$ of 0.5 $\mu$m or greater and less than 50 $\mu$m, a specific surface area of from 25 to 1000 m$^2$/g, and a sphericity of 0.85 or greater, and describes that a cosmetic having the porous cellulose particles blended has excellent tactile properties.

Methods of producing porous cellulose particles have also been studied. For example, Patent Document 3 describes that porous cellulose particles can be more easily produced without using a harmful solvent by a method of producing porous cellulose particles. The method includes: dissolving cellulose diacetate in a solvent to prepare a cellulose diacetate solution; dispersing the cellulose diacetate solution in a medium immiscible with the cellulose diacetate solution to give a dispersion system; cooling the dispersion system; adding a pore solvent to the cooled dispersion system and thereby precipitating cellulose diacetate particles; and saponifying the cellulose diacetate particles.

Patent Document 4 discloses a method of producing porous cellulose beads characterized in that the method includes bringing a cellulose dispersion prepared by mixing an alkaline aqueous solution in a predetermined temperature range with cellulose into contact with a coagulating solvent, and describes that porous cellulose beads having high mechanical strength can be produced without using highly toxic and corrosive auxiliary materials and without undergoing complicated steps which are industrially disadvantageous.

Patent Document 5 discloses that porous cross-linked cellulose beads having a pore structure suitable for antibody adsorption can be produced simply and efficiently without using highly toxic and corrosive auxiliary materials and without undergoing complicated steps that are industrially disadvantageous by a method including: dissolving cellulose in an alkali-containing aqueous solution at a predetermined temperature range to prepare a cellulose solution, then heating the cellulose solution, preparing an emulsion, and precipitating cellulose beads under predetermined conditions, followed by cross-linking of the precipitated cellulose beads.

Patent Document 6 discloses cellulose fine particles having a particle size of from 1 to 2500 $\mu$m, an average pore size of from 200 to 1000 $\mu$m, a specific surface area of from 500 to 800 m$^2$/g, a water content of from 86 to 93%, a pore volume of from 1.00 to 5.00 ml/g, and a porosity of from 90 to 95%, and a method of producing the same.

Citation List

Patent Literature

Patent Document 1: JP H02-84401 A
Patent Literature 2: WO 2020/004604 A
Patent Literature 3: WO 2015/029790 A
Patent Document 4: JP 2017-14528 A
Patent Document 5: JP 2021-161246 A
Patent Document 6: CN 101250267 A

Summary of Invention

Technical Problem

Functional particles to be blended in cosmetics are required to have excellent ability to impart tactile properties such as imparting a soft feel and being less gritty during application. Furthermore, when functional particles are porous, the particles are desired to be able to contain a functional substance, and when the cosmetic is applied to an object, the particles are desired to be deformed through application action and thereby allowing a sustained-release of the functional substance to the object.

The porous cellulose particles described in Patent Document 1 are crystalline cellulose of crystal form I and thus are hard particles, and it is believed that a user may feel squeakiness when the particles deform during application action. In addition, all of the porous cellulose particles disclosed in Examples have a pore volume of 1 mL/g or less, and it is believed that the inclusion of the functional substance is insufficient.

The porous cellulose particles described in Patent Document 2 are also aggregates of crystalline cellulose of crystal form I. The porous cellulose particles are described to have excellent tactile properties, but there is no description or suggestion about the inclusion of a functional substance by the particles and sustained release of a functional substance by the particles. Furthermore, the porous cellulose particles disclosed in Examples of Patent Document 2 each have a pore volume of 1 mL/g or less, and the inclusion of a functional substance is believed to be insufficient.

The porous cellulose particles described in Patent Document 3 and the porous cellulose beads described in Patent Document 4 are both used as a chromatography packing material or the like, and have a large average particle size, and presumably have poor tactile properties when used in cosmetics. In addition, porous cellulose particles that may deform through application action are not conceivable from these documents since the particles to be used for a chromatography packing material have preferably higher strength.

The porous cross-linked cellulose beads described in Patent Document 5 have been cross-linked and therefore are hard particles. In addition, these cellulose beads are also described as suitable for an adsorbent for chromatography, an affinity adsorbent, and the like, and have a porosity lower than that of particles for cosmetics applications designed to deform through application action.

The cellulose fine particles described in Patent Document 6 have a large specific surface area and a high water content.

The present invention relates to the provision of porous cellulose particles which can impart a soft feel when blended in a cosmetic, is less gritty during application, and are also excellent in the inclusion of a functional substance and tendency of the particles to deform.

Solution to Problem

The present inventors have found that the problems described above can be solved by porous cellulose particles having a compression modulus, a specific surface area, and a pore volume each within a predetermined range.

That is, the present invention relates to the following.

[1] Porous cellulose particles having a compression modulus of 50 MPa or less, a specific surface area of 100 $m^2$/g or greater and less than 500 $m^2$/g, and a pore volume of 1.5 mL/g or greater.

[2] A cosmetic containing the porous cellulose particles according to [1].

[3] A method of producing the porous cellulose particles according to [1], including the following steps (I) to step (V) in this order:

step (I): mixing raw material cellulose and an alkaline aqueous solution to prepare a cellulose aqueous solution;

step (II): mixing the cellulose aqueous solution with an organic solvent to prepare a cellulose emulsion;

step (III): mixing the cellulose emulsion and a non-cellulose-dissolving solvent to precipitate crude cellulose particles, thereby producing a suspension containing the crude cellulose particles;

step (IV): subjecting the suspension containing the crude cellulose particles to solid-liquid separation, and then cleaning the resultant crude cellulose wet particles to produce purified cellulose wet particles; and

step (V): subjecting the purified cellulose wet particles to a drying process to produce porous cellulose particles.

Advantageous Effects of Invention

According to the present invention, it is possible to provide porous cellulose particles capable of imparting a soft feel when blended in a cosmetic, being less gritty during application, and also being excellent in the inclusion of a functional substance and tendency of the particles to deform.

Brief Description of Drawings

FIG. 1 is an X-ray diffraction profile of raw material cellulose (Cellulose type I crystals) used in Examples.
FIG. 2 is an X-ray diffraction profile of the porous cellulose particles (Cellulose type II crystals) obtained in Example 1.
FIG. 3 is an X-ray diffraction profile of the porous cellulose particles (amorphous) obtained in Example 4.

Description of Embodiments

[Porous cellulose particles]

The porous cellulose particles of the present invention have a compression modulus of 50 MPa or less, a specific surface area of 100 $m^2$/g or greater and less than 500 $m^2$/g, and a pore volume of 1.5 mL/g or greater.

The porous cellulose particles of the present invention have the above-described configuration and are thereby capable of imparting a soft feel when blended in a cosmetic, being less gritty during application, and also being excellent in the inclusion of a functional substance and tendency of the particles to deform. The reason for this is not certain, but it is thought to be as follows.

It is believed that the porous cellulose particles having a compression modulus of a predetermined value or less can impart a soft feel, suppress grittiness during applying to an object, and easily deform by an application action to an object. It is also believed that the porous cellulose particles may exhibit improvement in functional substance inclusion, when the porous cellulose particles have a specific surface area of a predetermined value or greater and a pore volume of a predetermined value or greater.

The compression modulus of the porous cellulose particles is 50 MPa or less, preferably 40 MPa or less, more preferably 30 MPa or less, still more preferably 20 MPa or less, even more preferably 10 MPa or less, even more preferably 7.0 MPa or less, even more preferably 6.0 MPa or less, even more preferably 5.3 MPa or less, even more preferably 5.2 MPa or less, and even more preferably 5.0 MPa or less from the viewpoints of a soft feel, less grittiness during applying to an object, and improvement in deformation by an application action to an object. From the viewpoint of suppressing deformation in the process of producing porous cellulose particles, the compression modulus is preferably 1.0 MPa or greater, more preferably 2.0 MPa or greater, even more preferably 3.0 MPa or greater, even more preferably 4.0 MPa or greater, and even more preferably 4.9 MPa or greater. The compression modulus of the porous cellulose particles is 50 MPa or less, preferably 1.0 MPa or greater and 50 MPa or less, more preferably 1.0 MPa or greater and 40 MPa or less, still more preferably 1.0 MPa or greater and 30 MPa or less, even more preferably 1.0 MPa or greater and 20 MPa or less, even more preferably 2.0 MPa or greater and 10 MPa or less, even more preferably 2.0 MPa or greater and 7.0 MPa or less, even more preferably 3.0 MPa or greater and 6.0 MPa or less, even more preferably 4.0 MPa or greater and 5.3 MPa or less, even more preferably 4.0 MPa or greater and 5.2 MPa or less, and even more preferably 4.9 MPa or greater and 5.0 MPa or less.

The compression modulus is an apparent compression modulus of a single particle as measured by a micro compression testing machine, and specifically can be measured by the method described in Examples.

The compression modulus of the porous cellulose particles can be adjusted, for example, by altering the degree of polymerization of the raw material cellulose used in step (I) and the cellulose concentration in the aqueous solution prepared in step (I) in the method of producing the porous cellulose particles described later. Specifically, the resulting porous cellulose particles will have an increased compression modulus by using raw material cellulose having a high degree of polymerization or increasing the concentration of cellulose in the aqueous solution in step (I). Porous cellulose particles having a low compression modulus can be obtained by using raw material cellulose having a low degree of polymerization or reducing the cellulose concentration in the aqueous solution in step (I).

The median size ($D_{50}$) of the porous cellulose particles as measured by a dry method is preferably 75 $\mu$m or less, more preferably 70 $\mu$m or less, still more preferably 65 $\mu$m or less, even more preferably 55 $\mu$m or less, even more preferably 40 $\mu$m or less, and even more preferably 30 $\mu$m or less from the viewpoint of suppressing grittiness during applying the porous cellulose particles to an object. From the viewpoint of improving the deformation through application action to an object, the median size is preferably 5 $\mu$m or greater, more preferably 10 $\mu$m or greater, and still more preferably 15 $\mu$m or greater. The median size of the porous cellulose particles as measured by a dry method is preferably 5 $\mu$m or greater and 75 $\mu$m or less, more preferably 5 $\mu$m or greater and 70 $\mu$m or less, still more preferably 5 $\mu$m or greater and 65 $\mu$m or less, even more preferably 5 $\mu$m or greater and 55 $\mu$m or less, even more preferably 10 $\mu$m or greater and 40 $\mu$m or less, and even more preferably 15 $\mu$m or greater and 30 $\mu$m or less.

The median size of the porous cellulose particles as measured by a dry method is a 50% median size measured by a particle size distribution measuring device based on a laser diffraction/scattering method using dry particles as a measurement sample, and specifically, the median size can be measured by the method described in Examples.

The median size of the porous cellulose particles can be adjusted by, for example, altering the stirring speed during mixing the cellulose aqueous solution with an organic solvent in step (II) in the method of producing the porous cellulose particles described below. Specifically, higher stirring speed during mixing the cellulose aqueous solution with an organic solvent in step (II) will reduce the size of emulsion droplets of the resultant cellulose emulsion, and as a result, porous cellulose particles having a small median size can be obtained. Lower stirring speed will increase the size of emulsion droplets of the resultant cellulose emulsion, and as a result, porous cellulose particles having a large median size can be obtained.

The specific surface area of the porous cellulose particles is 100 $m^2/g$ or greater, preferably 110 $m^2/g$ or greater, more preferably 120 $m^2/g$ or greater, still more preferably 130 $m^2/$ g or greater, even more preferably 135 $m^2/g$ or greater, even more preferably 140 $m^2/g$ or greater, and even more preferably 144 $m^2/g$ or greater, from the viewpoint of improving the inclusion of a functional substance. From the viewpoint of suppressing deformation in the process of producing porous cellulose particles, the specific surface area is less than 500 $m^2/g$, preferably 200 $m^2/g$ or less, more preferably 180 $m^2/g$ or less, and still more preferably 150 $m^2/g$ or less. The specific surface area of the porous cellulose particles is 100 $m^2/g$ or greater and less than 500 $m^2/g$, preferably 100 $m^2/g$ or greater and 200 $m^2/g$ or less, more preferably 110 $m^2/g$ or greater and 180 $m^2/g$ or less, still more preferably 120 $m^2/g$ or greater and 180 $m^2/g$ or less, even more preferably 130 $m^2/g$ or greater and 150 $m^2/g$ or less, even more preferably 135 $m^2/g$ or greater and 150 $m^2/g$ or less, even more preferably 140 $m^2/g$ or greater and 150 $m^2/g$ or less, and even more preferably 144 $m^2/g$ or greater and 150 $m^2/g$ or less.

The specific surface area is a value obtained as follows: the mercury intrusion porosimetry is employed to determine the sum of the surface areas of the microscopic surfaces inside the porous cellulose particles and the surface areas of the particle surfaces; and this sum is divided to normalize by the mass of the particles. Specifically, the specific surface area can be measured by the method described in Examples.

The specific surface area of the porous cellulose particles can be controlled by, for example, the surface tension of a dispersion medium (an organic solvent) used in the dispersion medium replacement carried out as necessary in step (IV) and the selection of drying method in step (V) in the method of producing the porous cellulose particles described below. Specifically, when the surface tension of an organic solvent used for the dispersion medium replacement in step (IV) is small, the capillary force caused by the volatilization of the organic solvent is small, the shrinkage of the cellulose particles during drying can be suppressed, and porous cellulose particles having a large specific surface area can be obtained. Alternatively, in step (V), using a drying method which does not involve capillary force due to the surface tension of an organic solvent, such as freeze-drying, the shrinkage of the cellulose particles during drying can be suppressed, and porous cellulose particles having a large specific surface area can be obtained.

The pore volume of the porous cellulose particles is 1.5 mL/g or greater, preferably 2.0 mL/g or greater, more preferably 2.5 mL/g or greater, still more preferably 3.0 mL/g or greater, even more preferably 3.5 mL/g or greater, and even more preferably 4.0 mL/g or greater from the viewpoint of improving the inclusion of a functional substance. From the viewpoint of suppressing deformation in the process of producing porous cellulose particles, the pore volume is preferably 8.0 mL/g or less, more preferably 7.0 mL/g or less, still more preferably 6.0 mL/g or less, and even more preferably 5.0 mL/g or less. The pore volume of the porous cellulose particles is 1.5 mL/g or greater, preferably 1.5 mL/g or greater and 8.0 mL/g or less, more preferably 2.0 mL/g or greater and 7.0 mL/g or less, still more preferably 2.5 mL/g or greater and 6.0 mL/g or less, even more preferably 3.0 mL/g or greater and 5.0 mL/g or less, even more preferably 3.5 mL/g or greater and 5.0 mL/g or less, and even more preferably 4.0 mL/g or greater and 5.0 mL/g or less.

The pore volume is a value obtained as follows: the mercury intrusion porosimetry is employed to determine the total volume of mercury that has been incorporated into the pores inside the porous cellulose particles and the gaps between the particles; and this total volume is divided by the mass of the particles for normalization. Specifically, the pore volume can be measured by the method described in Examples.

The pore volume of the porous cellulose particles can be adjusted, for example, by adjusting the size of emulsion droplets of the cellulose emulsion obtained in step (II) as well as by the surface tension of a dispersion medium (an organic solvent) used for the dispersion medium replacement carried out as necessary in step (IV) and the selection of drying method in step (V) in the method of producing the porous cellulose particles described below. Specifically, when the emulsion droplet size of the cellulose emulsion is small, the median size and pore volume of the resultant porous cellulose particles are also small.

The porous cellulose particles of the present invention are preferably porous cellulose particles without chemical modification from the viewpoint of environmental friendliness. As used herein "without chemical modification" means that (1) substantially no substituents have been introduced in a hydroxy group in cellulose that constitutes the porous cellulose particles, and that (2) the surface of the porous cellulose particles is not coated with a surface treatment agent. The amounts of substituents introduced in the hydroxy group in the cellulose that constitutes the porous cellulose particles is preferably 0.5 mol% or less, more preferably 0.1 mol% or less, and still more preferably 0 mol% of the total hydroxy groups.

The porous cellulose particles are preferably non-crosslinked particles from the viewpoint of imparting a soft feel, from the viewpoint of improving the inclusion of a functional substance, and from the viewpoint of improving tendency to deform through application action to an object. The non-crosslinked particles mean particles which are produced without an intentional crosslinking reaction and have substantially no crosslinked structure.

The surface pore size of the porous cellulose particles is preferably 50 nm or greater, more preferably 100 nm or greater, and still more preferably 200 nm or greater from the viewpoint of improving the inclusion of a functional substance, and is preferably 800 nm or less, more preferably 600 nm or less, and still more preferably 500 nm or less from the viewpoint of having high particle strength and maintaining the particle shape. The surface pore size of the porous cellulose particles is preferably 50 nm or greater and 800 nm or less, more preferably 100 nm or greater and 600 nm or less, and still more preferably 200 nm or greater and 500 nm or less.

The surface pore size is determined by mercury intrusion porosimetry, and specifically, can be measured by a method described in Examples.

The sphericity of the porous cellulose particles is preferably 60% or greater, more preferably 70% or greater, and still more preferably 80% or greater, from the viewpoint of improving the sense of touch when applied to an object and from the viewpoint of imparting appropriate tendency of the particles to deform. The upper limit of the sphericity is 100%, and may be 90% or less. Note that, the sphericity of the porous cellulose particles is a value defined by the following formula, and can be determined by measurement with a dynamic image analyzer CAMSIZER X2 (available from MICROTRAC MRB). Specifically, the sphericity can be measured by the method described in Examples.

$$\text{Sphericity (\%)} = 4\pi \times \text{area of particle (m}^2)/(\text{outer periphery of particle (m)})^2 \times 100$$

The cellulose that constitutes the porous cellulose particles is preferably not crystalline cellulose of cellulose type I but crystalline cellulose of cellulose type II or amorphous cellulose from the viewpoint of imparting a soft feel and from the viewpoint of improving tendency to deform through application action to an object.

The crystal form of the cellulose that constitutes the porous cellulose particles can be identified from a diffraction angle and a diffraction intensity by an X-ray diffraction method. The crystalline cellulose of cellulose type II exhibits a diffraction peak attributed to (11-0) plane at a diffraction angle $2\theta = 12.5°$ and a diffraction peak attributed to (110) plane at a diffraction angle $2\theta = 20.0°$ and can be easily distinguished from the crystalline cellulose of cellulose type I. The crystallinity of cellulose type II is defined by the following equation, but its value is not particularly limited.

$$\text{Crystallinity of cellulose type II (\%)} = [(I_{20.0} - I_{15.0})/I_{20.0}] \times 100$$

(wherein $I_{20.0}$ is a diffraction intensity of a lattice plane (110 plane) (diffraction angle $2\theta = 20.0°$) in X-ray diffraction, and $I_{15.0}$ is a diffraction intensity of an amorphous portion (diffraction angle $2\theta = 15.0°$).

The crystallinity of cellulose type II can be measured by an X-ray diffraction method, specifically by the method described in Examples.

The crystal form (the crystallinity of cellulose type II) of the porous cellulose particles can be adjusted, for example, depending on the type of solvent contained in purified cellulose wet particles when performing a drying process in step (V) in the method of producing the porous cellulose particles described below. Although the reason is not clear, but the crystallinity is increased when the drying process is performed in step (V) in a state where the purified cellulose wet particles contain an aqueous solvent, and the crystallinity decreases when the drying process is performed in step (V) in a state where the purified wet cellulose particles contain a non-aqueous solvent.

The physical properties of the porous cellulose particles can be adjusted, for example, by choosing suitable conditions for producing cellulose particles, selecting the type of raw material cellulose used for producing suitable cellulose particles, and the like, specifically as stated above.

The porous cellulose particles preferably contain smaller amounts of compounds besides cellulose, such as impurities contained in the raw material cellulose, a smaller amount of a solvent used during production, and smaller amounts of additives, and the like. That is, the content of cellulose in the porous cellulose particles is preferably 95 mass% or greater, more preferably 99 mass% or greater, and still more preferably substantially 100 mass%.

[Method of producing porous cellulose particles]

The porous cellulose particles of the present invention can be preferably produced by a production method including the following steps (I) to step (V) in this order.

step (I): mixing raw material cellulose and an alkaline aqueous solution to prepare a cellulose aqueous solution;
step (II): mixing the cellulose aqueous solution with an organic solvent to prepare a cellulose emulsion;
step (III): mixing the cellulose emulsion and a non-cellulose-dissolving solvent to precipitate crude cellulose particles, thereby producing a suspension containing the crude cellulose particles;
step (IV): subjecting the suspension containing the crude cellulose particles to solid-liquid separation, and then cleaning the resultant crude cellulose wet particles to produce purified cellulose wet particles; and
step (V): subjecting the purified cellulose wet particles to a drying process to produce porous cellulose particles.

According to the production method described above, porous cellulose particles having the physical properties described above can be easily produced.

<Step (I)>

In step (I), raw material cellulose and an alkaline aqueous solution are mixed to prepare a cellulose aqueous solution. Unlike cellulose suspension, the cellulose aqueous solution prepared in step (I) is a solution of cellulose dissolved in an alkaline aqueous solution. Here, the state of "cellulose dissolution" means a state where the cellulose aqueous solution is visually transparent. Note that, a part of cellulose may be in a dispersed state.

Preparing a cellulose aqueous solution in step (I) and subjecting this aqueous solution to step (II) and the subsequent steps presumably facilitates the control of the morphology inside the cellulose particles and the production of the porous cellulose particles having desired physical properties.

(Raw material cellulose)

From the viewpoint of environmental friendliness, the raw material cellulose used in step (I) is preferably chemically pure cellulose without chemical modification. As the raw material cellulose, there may be used various cellulose-containing raw materials including: wood such as various wood chips, pruned branches of various trees, thinned wood, branch wood, wooden building waste, and wooden factory waste; pulp such as wood pulp produced from wood and cotton linter pulp obtained from fibers around cotton seeds; paper such as newspaper, cardboard, magazine, and high-quality paper; plant stems and leaves such as rice straw and corn stalk; and plant shells such as chaff, palm shell, and coconut shell. Among these, from the viewpoint of cellulose purity in the raw material cellulose, degree of polymerization of cellulose, and availability, preferred are wood such as various wood chips, pruned branches of various trees, thinned wood, branch wood, building waste, and factory waste; and pulp such as wood pulp produced from wood and cotton linter pulp obtained from fibers around cotton seeds.

Examples of form of the raw material cellulose include a powder form, a sheet form, and a flocculent form. Among them, the raw material cellulose is preferably in the form of powder from the viewpoint of excellent solubility in an alkaline aqueous solution.

The degree of polymerization of the raw material cellulose is preferably 10 or greater, more preferably 50 or greater, still more preferably 100 or greater, and even more preferably 150 or greater from the viewpoint of the strength of resulting porous cellulose particles, and is preferably 1000 or less, more preferably 500 or less, and still more preferably 300 or less from the viewpoint of enhancing the solubility in an alkaline aqueous solution. The degree of polymerization of the raw material cellulose is preferably 10 or greater and 1000 or less, more preferably 50 or greater and 500 or less, still more preferably 100 or greater and 500 or less, and even more preferably 150 or greater and 300 or less.

The degree of polymerization of the raw material cellulose is typically controlled by the acid hydrolysis condition of raw material pulp. For example, longer acid hydrolysis time will provide raw material cellulose having a low degree of polymerization.

As the raw material cellulose, both crystalline cellulose and amorphous cellulose can be used, but from the viewpoints of producing porous cellulose particles having desired physical properties and of availability, crystalline cellulose is preferable, and crystalline cellulose of cellulose type I is more preferable.

When the raw material cellulose is in a powder form, the median size of the raw material cellulose is preferably 10 μm or greater, and more preferably 20 μm or greater from the viewpoint of improving handleability, and is preferably 500 μm or less, more preferably 300 μm or less, still more preferably 200 μm or less, and even more preferably 150 μm or less from the viewpoint of enhancing solubility in an alkaline aqueous solution.

The median size of the raw material cellulose can be measured by the same method as described above.

(Alkaline aqueous solution)

The alkaline aqueous solution used in step (I) is not particularly limited as long as it shows alkalinity and is capable of dissolving cellulose. Here, the phrase "capable of dissolving cellulose" means, for example, that cellulose is mixed into an alkaline aqueous solution in such an amount that the concentration becomes 4 mass% in the solution, and dissolution can be visually confirmed.

As the alkaline compound used for the alkaline aqueous solution, any of inorganic alkaline compounds and organic alkaline compounds can be used, and examples thereof include: alkali metal hydroxides such as sodium hydroxide, potassium hydroxide, and lithium hydroxide; ammonia; and tertiary amines such as trimethylamine and triethylamine. Among them, from the viewpoint of availability and economic efficiency, an alkali metal hydroxide is preferable, one or more selected from the group consisting of sodium hydroxide and potassium hydroxide are more preferable, and sodium hydroxide is still more preferable.

One of the alkaline compounds can be used alone, or two or more types thereof may be used in combination.

The concentration of the alkaline compound in the alkaline aqueous solution is preferably 1 mass% or greater, more preferably 2 mass% or greater, and still more preferably 3 mass% or greater, and preferably 40 mass% or less, more preferably 30 mass% or less, and still more preferably 25 mass% or less, from the viewpoints of improving the solubility of the raw material cellulose and the stability of the resulting cellulose aqueous solution. The concentration of the alkaline compound in the alkaline aqueous solution is preferably 1 mass% or greater and 40 mass% or less, more preferably 2 mass% or greater and 30 mass% or less, and still more preferably 3 mass% or greater and 25 mass% or less.

In step (I), from the viewpoint of improving the production efficiency and from the viewpoint of improving the solubility of the raw material cellulose and the stability of the resulting cellulose aqueous solution, the alkaline aqueous solutions having different concentrations may be mixed with the raw material cellulose in multiple times.

Specifically, in step (I), the cellulose aqueous solution is preferably prepared in a manner that the raw material cellulose is mixed with an alkaline aqueous solution A containing an alkaline compound at a concentration of 1 mass% or greater and 10 mass% or less, and then an alkaline aqueous solution B containing an alkaline compound at a concentration of more than 10 mass% and 40 mass% or less is added thereto and mixed.

The concentration of the alkaline compound in the alkaline aqueous solution A is more preferably 2 mass% or greater and 8 mass% or less, and still more preferably 2 mass% or greater and 5 mass% or less.

The concentration of the alkaline compound in the alkaline aqueous solution B is more preferably 15 mass% or greater and 30 mass% or less, and still more preferably 20 mass% or greater and 25 mass% or less.

When the alkaline aqueous solution A and the alkaline aqueous solution B are used in step (I), the ratio therebetween is not particularly limited, but the mass ratio (A/B) of the alkaline aqueous solution A to the alkaline aqueous solution B is preferably 1 or greater and 10 or less, more preferably 2 or greater and 8 or less, and still more preferably 3 or greater and 6 or less, from the viewpoints of improving the production efficiency and improving the stability of the resulting cellulose aqueous solution.

The mixing of the raw material cellulose and the alkaline aqueous solution in step (I) can be performed by adding the raw material cellulose to the alkaline aqueous solution and stirring the mixture with a known apparatus.

The temperature during mixing of the raw material cellulose and the alkaline aqueous solution is preferably 10°C or lower, more preferably 5°C or lower, and still more preferably 0°C or lower, from the viewpoint of uniformly dispersing and efficiently dissolving the raw material cellulose. From the viewpoint of improving the solubility of cellulose without freezing, the temperature is preferably -20°C or higher, more preferably -10°C or higher, and still more preferably -5°C or higher. The temperature during mixing of the raw material cellulose and the alkaline aqueous solution is preferably -20°C or higher and 10°C or lower, more preferably -10°C or higher and 5°C or lower, and still more preferably -5°C or higher and 0°C or lower.

When using the alkaline aqueous solution A and the alkaline aqueous solution B, it is preferable that the raw material cellulose is added to the alkaline aqueous solution A and mixed with stirring, then the temperature of the mixture is adjusted to the above-mentioned range, and thereafter the alkaline aqueous solution B is added thereto and mixed.

The stirring time depends on the production scale, the concentration of the alkaline compound in the alkaline aqueous solution, and the temperature, and is appropriately set, and thus is not particularly limited, typically stirring is continued until dissolution of the raw material cellulose is visually confirmed.

The cellulose concentration in the cellulose aqueous solution obtained in step (I) is preferably 0.5 mass% or greater, more preferably 1 mass% or greater, and still more preferably 2 mass% or greater, from the viewpoint of enhancement of the strength of the resulting porous cellulose particles. The cellulose concentration is preferably 15 mass% or less, more preferably 10 mass% or less, still more preferably 8 mass% or less, and even more preferably 6 mass% or less, from the viewpoint of achieving a viscosity at which the cellulose emulsion is easily prepared when the cellulose aqueous solution is subjected to step (II). The cellulose concentration in the cellulose aqueous solution obtained in step (I) is preferably 0.5 mass% or greater and 15 mass% or less, more preferably 1 mass% or greater and 10 mass% or less, still more preferably 1 mass% or greater and 8 mass% or less, and even more preferably 2 mass% or greater and 6 mass% or less.

The concentration of the alkaline compound in the cellulose aqueous solution obtained in step (I) is preferably 0.5 mass% or greater, more preferably 1 mass% or greater, still more preferably 2 mass% or greater, even more preferably 3 mass% or greater, and even more preferably 5 mass% or greater, and is preferably 15 mass% or less, more preferably 12 mass% or less, and still more preferably 10 mass% or less, from the viewpoint of improving the solubility of raw material cellulose and the stability of the resulting cellulose aqueous solution. The concentration of the alkaline compound in the cellulose aqueous solution obtained in step (I) is preferably 0.5 mass% or greater and 15 mass% or less, more preferably 1 mass% or greater and 15 mass% or less, still more preferably 2 mass% or greater and 12 mass% or less, even more preferably 3 mass% or greater and 10 mass% or less, and even more preferably 5 mass% or greater and 10 mass% or less.

<Step (II)>

**In** step (II), the cellulose aqueous solution obtained in step (I) is mixed with an organic solvent to prepare a cellulose emulsion. By step (II), a water-in-oil type cellulose emulsion can be prepared, with which porous cellulose particles having a desired median size can be produced.

The organic solvent is not particularly limited as long as it is a water-immiscible organic solvent that can be mixed with the cellulose aqueous solution to prepare a cellulose emulsion.

Preferred examples of the organic solvent used in step (II) include hydrocarbon solvents, ester-based solvents, and halogen-based solvents.

Examples of the hydrocarbon solvent include a chain aliphatic hydrocarbon, an alicyclic hydrocarbon, and an aromatic hydrocarbon.

The number of carbon atoms in the chain aliphatic hydrocarbon is preferably 6 or more, more preferably 8 or more, and preferably 18 or less, more preferably 12 or less. The chain aliphatic hydrocarbon may be either a linear aliphatic hydrocarbon or a branched aliphatic hydrocarbon.

The number of carbon atoms in the alicyclic hydrocarbon and the aromatic hydrocarbon is preferably 6 or more and 18 or less, and more preferably 6 or more and 12 or less.

Specific examples of the hydrocarbon solvent include n-pentane, n-hexane, n-heptane, n-octane, isooctane, n-decane, isodecane, n-dodecane, isododecane, tetradecane, hexadecane, octadecane, cyclohexane, methylcyclohexane, cycloheptane, methylcycloheptane, toluene, and xylene.

The ester-based solvent is preferably an ester having from 4 to 10 carbon atoms, and examples thereof include ethyl acetate and butyl acetate.

Examples of the halogen-based solvent include dichloromethane, dichloroethane, and dichlorobenzene.

One of these organic solvents may be used alone, or two or more types thereof may be mixed and used.

The organic solvent is preferably a hydrocarbon solvent, more preferably a chain aliphatic hydrocarbon, still more preferably one or more selected from the group consisting of n-pentane, n-hexane, n-heptane, n-octane, isooctane, decane, isodecane, dodecane, isododecane, tetradecane, hexadecane, and octadecane, and even more preferably one or more selected from the group consisting of n-octane, isooctane, n-decane, isodecane, n-dodecane, and isododecane, from the viewpoint of enabling to easily prepare a water-in-oil type cellulose emulsion.

In step (II), the amount of the organic solvent to be mixed with the cellulose aqueous solution is preferably 80 parts by mass or greater, more preferably 100 parts by mass or greater, and still more preferably 120 parts by mass or greater per 100 parts by mass of the cellulose aqueous solution from the viewpoint of improving the emulsion stability of the water-in-oil type cellulose emulsion, and is preferably 1000 parts by mass or less, more preferably 800 parts by mass or less, still more preferably 500 parts by mass or less, and even more preferably 300 parts by mass or less from the viewpoint of easily obtaining the porous cellulose particles having a desired median size. In step (II), the amount of the organic solvent to be mixed with the cellulose aqueous solution is preferably 80 parts by mass or greater and 1000 parts by mass or less, more preferably 100 parts by mass or greater and 800 parts by mass or less, still more preferably 120 parts by mass or greater and 500 parts by mass or less, and even more preferably 120 parts by mass or greater and 300 parts by mass or less per 100 parts by mass of the cellulose aqueous solution.

In step (II), besides the cellulose aqueous solution and the organic solvent, an emulsifier is preferably further mixed from the viewpoint of improving the emulsion stability of the water-in-oil type cellulose emulsion.

Examples of the emulsifier include nonionic surfactants, anionic surfactants, cationic surfactants, and amphoteric surfactants. Among them, nonionic surfactants are preferable from the viewpoint of improving emulsifiability of the water-in-oil type cellulose emulsion.

From the viewpoint of improving the emulsion stability of the water-in-oil type cellulose emulsion, the hydrophile-lipophile balance (HLB) of a nonionic surfactant to be used as the emulsifier is preferably 1 or greater and 10 or less, more preferably 1 or greater and 8 or less, still more preferably 1 or greater and 6 or less, even more preferably 1 or greater and 5 or less, even more preferably 1 or greater and 4 or less, and even more preferably 1 or greater and 3 or less.

Here, the HLB is an index representing a ratio of relative affinity of a surfactant to both liquids in an oil-water system, and can be determined by the following formula of the Griffin method (J. Soc. Cosm. Chem., 1954, 5: 249-256).

$$\text{HLB} = 20 \times [(\text{molecular weight of hydrophilic group contained in surfactant})/(\text{molecular weight of surfactant})]$$

Examples of the hydrophilic group contained in the surfactant include a hydroxy group and an ethyleneoxy group.

Note that, the HLB of two or more nonionic surfactants can be determined as a weighted average obtained by summation of the HLB of each nonionic surfactant multiplied by the mass fraction of the respective nonionic surfactant (i.e., a value of the mass of each nonionic surfactant divided by the total mass of the nonionic surfactants).

Examples of the nonionic surfactant used as the emulsifier include a sorbitan fatty acid ester, a polyoxyethylene sorbitan fatty acid ester, a polyoxyethylene alkyl ether, a polyoxyethylene glycerin fatty acid ester, a polyoxyethylene sorbitol fatty acid ester, a polyoxyethylene hydrogenated castor oil, a polyglyceryl fatty acid ester, a sucrose fatty acid ester, and a polyether-modified silicone, and one of these can be used alone, or two or more thereof may be used in combination.

The number of carbon atoms in the fatty acid in the sorbitan fatty acid ester, the polyoxyethylene sorbitan fatty acid ester, the polyoxyethylene glycerin fatty acid ester, the polyoxyethylene sorbitol fatty acid ester, the polyglyceryl fatty acid ester, or the sucrose fatty acid ester, and of the alkyl group in the polyoxyethylene alkyl ether is preferably 12 or more, more preferably 16 or more, and still more preferably 18 or more, and preferably 24 or less, and more preferably 22 or less, from the viewpoint of adjusting the **HLB** preferably to be within the above-mentioned range.

Examples of the sorbitan fatty acid ester include sorbitan monooleate, sorbitan monostearate, sorbitan sesquioleate, sorbitan cocoate, sorbitan monopalmitate, sorbitan tristearate, and sorbitan trioleate.

Examples of the polyoxyethylene sorbitan fatty acid ester include polyoxyethylene sorbitan monooleate and polyoxyethylene sorbitan trioleate.

Examples of the polyoxyethylene alkyl ether include polyoxyethylene lauryl ether, polyoxyethylene oleyl ether, and polyoxyethylene stearyl ether.

Examples of the polyoxyethylene glycerin fatty acid ester include polyoxyethylene glyceryl monooleate.

Examples of the polyoxyethylene sorbitol fatty acid ester include polyoxyethylene sorbitol tetraoleate.

Examples of the sucrose fatty acid ester include sucrose palmitate, sucrose oleate, sucrose stearate, sucrose erucate, and sucrose behenate.

Among these, from the viewpoint of further improving the emulsion stability of the water-in-oil type cellulose emulsion, the nonionic surfactant used as the emulsifier is preferably one or more selected from the group consisting of a sorbitan fatty acid ester, a polyoxyethylene alkyl ether, a sucrose fatty acid ester, and a polyether-modified silicone, more preferably a sucrose fatty acid ester, still more preferably one or more selected from the group consisting of sucrose palmitate, sucrose oleate, sucrose stearate, sucrose erucate, and sucrose behenate, and even more preferably one or more selected from the group consisting of sucrose erucate and sucrose behenate.

When the emulsifier is further mixed in step (II), the mixing amount of the emulsifier is preferably 0.1 parts by mass or greater, more preferably 0.5 parts by mass or greater, and still more preferably 1.0 parts by mass or greater, and is preferably 20 parts by mass or less, more preferably 10 parts by mass or less, and still more preferably 5.0 parts by mass or less per 100 parts by mass of the organic solvent, from the viewpoint of further improving the emulsion stability of the water-in-oil type cellulose emulsion. The mixing amount of the emulsifier is preferably 0.1 parts by mass or greater and 20 parts by mass or less, more preferably 0.5 parts by mass or greater and 10 parts by mass or less, and still more preferably 1.0 parts by mass or greater and 5.0 parts by mass or less per 100 parts by mass of the organic solvent.

The emulsifier may be added to either the cellulose aqueous solution or the organic solvent before mixing, or may be added after mixing the cellulose aqueous solution and the organic solvent.

The preparation of the cellulose emulsion can be made, for example, by adding an organic solvent and an emulsifier to the cellulose aqueous solution and stirring the mixture with a known mixer such as a homo-mixer or a highspeed emulsifying disperser.

The temperature during mixing the cellulose aqueous solution and the organic solvent is preferably 20°C or lower, more preferably 10°C or lower, and still more preferably 5°C or lower from the viewpoint of further improving the emulsion stability of the water-in-oil type cellulose emulsion. From the viewpoint of preparing a water-in-oil type cellulose emulsion without freezing, the temperature is preferably -20°C or higher, more preferably -10°C or higher, and still more preferably -5°C or higher. The temperature during mixing of the cellulose aqueous solution and the organic solvent is preferably -20°C or higher and 20°C or lower, more preferably -10°C or higher and 10°C or lower, and still more preferably -5°C or higher and 5°C or lower.

The stirring speed during mixing of the cellulose aqueous solution and the organic solvent is appropriately selected according to production scale, apparatus used, the viscosity of the cellulose emulsion, and the like, but from the viewpoints of controlling the emulsion droplet size and producing porous cellulose particles having a desired median size, the stirring speed is preferably 1000 rpm or greater, more preferably 3000 rpm or greater, still more preferably 5000 rpm or greater, and even more preferably 5500 rpm or greater, and is preferably 15000 rpm or less, more preferably 12000 rpm or less, still more preferably 10000 rpm or less, and even more preferably 8500 rpm or less. The stirring speed during mixing of the cellulose aqueous solution and the organic solvent is preferably 1000 rpm or greater and 15000 rpm or less, more preferably 3000 rpm or greater and 12000 rpm or less, still more preferably 5000 rpm or greater and 10000 rpm or less, and even more preferably 5500 rpm or greater and 8500 rpm or less.

The mixing time of the cellulose aqueous solution and the organic solvent is appropriately selected according to production scale, apparatus used, the viscosity of the cellulose emulsion, and the like.

<Step (III)>

In step (III), the cellulose emulsion obtained in step (II) is mixed with a non-cellulose-dissolving solvent to precipitate crude cellulose particles, thereby producing a suspension containing crude cellulose particles.

The non-cellulose-dissolving solvent is a so-called non-solvent having no ability to dissolve cellulose, and is a solvent miscible with the alkaline aqueous solution and the organic solvent. By mixing the solvent and the cellulose emulsion, the non-cellulose-dissolving solvent is allowed to flow into the cellulose and thereby causes the organic solvent in the cellulose to flow out, and a phase-separated structure is created. This makes it possible to control the morphology inside the cellulose particles, to allow the cellulose to coagulate in a state where a desired porous structure is formed, and to precipitate the cellulose as particles.

The non-cellulose-dissolving solvent is preferably an alcohol-based solvent, and more preferably an alcohol having 4 or less carbon atoms.

Examples of the alcohol used as the non-cellulose-dissolving solvent include methanol, ethanol, 1-propanol, 2-propanol, 1-butanol, 2-butanol, 2-methyl-1-propanol, and tert-butyl alcohol, and one of these alcohols can be used alone, or two or more thereof may be used in combination.

Among these, the non-cellulose-dissolving solvent is preferably one or more selected from the group consisting of ethanol, 2-propanol, 1-butanol, 2-butanol, and 2-methyl-1-propanol, and more preferably ethanol, from the viewpoint of easily precipitating the crude cellulose particles and from the viewpoint of controlling the morphology inside the cellulose particles and thereby producing porous cellulose particles having desired physical properties.

The mixing amount of the non-cellulose-dissolving solvent is preferably 50 parts by mass or greater, more preferably 100 parts by mass or greater, and still more preferably 200 parts by mass or greater, and is preferably 1000 parts by mass or less, more preferably 500 parts by mass or less, and still more preferably 400 parts by mass or less per 100 parts by mass of the cellulose emulsion from the viewpoint of easily precipitating the crude cellulose particles, from the viewpoints of controlling the morphology inside the cellulose particles and thereby obtaining porous cellulose particles having desired physical properties, and from the viewpoint of maintaining the stability of the emulsion. The mixing amount of the non-cellulose-dissolving solvent is preferably 50 parts by mass or greater and 1000 parts by mass or less, more preferably 100 parts by mass or greater and 500 parts by mass or less, and still more preferably 200 parts by mass or greater and 400 parts by mass or less per 100 parts by mass of the cellulose emulsion.

In step (III), an acid is preferably further mixed from the viewpoint of neutralizing the alkaline compound remaining in the crude cellulose particles.

The acid may be either an inorganic acid or an organic acid, but is preferably an organic acid, and more preferably a carboxylic acid having 4 or less carbon atoms from the viewpoint of solubility in the cellulose emulsion and in the non-cellulose-dissolving solvent.

Examples of the carboxylic acid having 4 or less carbon atoms include a monocarboxylic acid, a dicarboxylic acid, and a hydroxycarboxylic acid, each having 4 or less carbon atoms, such as formic acid, acetic acid, propionic acid, butyric acid, lactic acid, citric acid, malic acid, and succinic acid. Among these, from the viewpoint of solubility in the cellulose emulsion and in the non-cellulose-dissolving solvent, one or more selected from the group consisting of acetic acid, lactic acid, malic acid, and succinic acid are preferable, and acetic acid is more preferable.

When an acid is used in step (III), the mixing amount of the acid is preferably 1.0 equivalent or greater, more preferably 1.2 equivalents or greater, and still more preferably 1.4 equivalents or greater with respect to the alkaline compound used in step (I) from the viewpoint of neutralizing the alkaline compound remaining in the crude cellulose particles, and is preferably 3.0 equivalents or less, more preferably 2.0 equivalents or less, and still more preferably 1.8 equivalents or less from the viewpoint of economic efficiency. The mixing amount of the acid in step (III) is preferably 1.0 equivalent or greater and 3.0 equivalents or less, more preferably 1.2 equivalents or greater and 2.0 equivalents or less, and still more preferably 1.4 equivalents or greater and 1.8 equivalents or less with respect to the alkaline compound used in step (I).

The mixing of the cellulose emulsion and the non-cellulose-dissolving solvent can be performed, for example, by adding the cellulose emulsion to the non-cellulose-dissolving solvent and stirring the mixture with a known apparatus. When the cellulose emulsion is added to the non-cellulose-dissolving solvent, the cellulose emulsion is preferably added and mixed to the non-cellulose-dissolving solvent under stirring in order to prevent emulsion droplets from combining with each other.

The temperature during mixing of the cellulose emulsion and the non-cellulose-dissolving solvent is preferably 0°C or higher, more preferably 5°C or higher, and still more preferably 15°C or higher, and is preferably 50°C or lower, more preferably 40°C or lower, and still more preferably 30°C or lower. The temperature during mixing of the cellulose emulsion and the non-cellulose-dissolving solvent is preferably 0°C or higher and 50°C or lower, more preferably 5°C or higher and 40°C or lower, and still more preferably 15°C or higher and 30°C or lower.

When the acid is mixed in step (III), the acid may be mixed simultaneously with mixing of the cellulose emulsion and the non-cellulose-dissolving solvent, or may be mixed after mixing of the cellulose emulsion and the non-cellulose-dissolving solvent. From the viewpoint of efficiently neutralizing the alkaline compound remaining in the crude cellulose particles (including a neutralization salt and the emulsifier as impurities), the acid is preferably mixed after mixing of the cellulose emulsion and the non-cellulose-dissolving solvent.

The stirring speed during mixing of the cellulose emulsion and the non-cellulose-dissolving solvent is appropriately set depending on production scale and temperature, but is preferably 100 rpm or greater, more preferably 200 rpm or greater, and preferably 2000 rpm or less, more preferably 1500 rpm or less, still more preferably 1000 rpm or less, even more preferably 800 rpm or less from the viewpoint of sufficiently precipitating the crude cellulose particles and from the viewpoint of controlling the morphology inside the cellulose particles and thereby producing porous cellulose particles having desired physical properties. The stirring speed during mixing of the cellulose emulsion and the non-cellulose-dissolving solvent is preferably 100 rpm or greater and 2000 rpm or less, more preferably 200 rpm or greater and 1500 rpm or less, still more preferably 200 rpm or greater and 1000 rpm or less, and even more preferably 200 rpm or greater and 800 rpm or less.

The stirring time during mixing of the cellulose emulsion and the non-cellulose-dissolving solvent is appropriately set depending on the production scale and temperature, and is usually 0.2 hours or greater and 12 hours or less, and preferably 0.5 hours or greater and 6 hours or less from the viewpoint of sufficiently precipitating the crude cellulose particles and from the viewpoint of controlling the morphology inside the cellulose particles and thereby obtaining porous cellulose particles having desired physical properties.

<Step (IV)>

In step (IV), the suspension containing the crude cellulose particles obtained in step (III) is subjected to solid-liquid separation, and then the resultant crude cellulose wet particles are subjected to a cleaning process to produce purified cellulose wet particles.

The solid-liquid separation of the suspension containing the crude cellulose particles can be performed by centrifugation, filtration, decantation, or a combination thereof.

Subsequently, in order to remove impurities such as the organic solvent and the emulsifier used in step (II) and the neutralization salt produced in step (III) from the crude cellulose wet particles obtained after the solid-liquid separation, the crude cellulose wet particles are subjected to a cleaning process. The cleaning process of the crude cellulose wet particles can be performed using water, an organic solvent, or a combination thereof. An organic solvent is preferably used to remove hydrophobic impurities such as the organic solvent and the emulsifier used in step (II), and water is preferably used to remove water-soluble impurities such as the neutralization salt.

As the organic solvent used for the cleaning process of the crude cellulose wet particles in step (IV), a solvent that is capable of dissolving the organic solvent and the emulsifier used in step (II) and is easy to dry is preferable, and examples thereof include a ketone-based solvent having 6 or less carbon atoms such as acetone and methyl isobutyl ketone, and an alcohol-based solvent having 6 or less carbon atoms such as ethanol and 2-propanol.

When drying by reduced-pressure drying is conducted, after the cleaning process, dispersion medium replacement is preferably carried out in which the cellulose particles after the cleaning process are further dispersed in a dispersion medium from the viewpoint of suppressing shrinkage of the resulting porous cellulose particles during drying.

From the viewpoint of suppressing shrinkage of the resulting porous cellulose particles during drying, the dispersion medium to be used for the dispersion medium replacement is preferably an organic solvent having a low surface tension, and is preferably an organic solvent having a surface tension of 20 mN/m or less, more preferably 18 mN/m or less at 25°C.

The surface tension is measured at 25°C with an automatic surface tensiometer (K100 available from KRUSS GmbH).

Examples of the organic solvent having a low surface tension include: an aliphatic hydrocarbon having 7 or less carbon atoms such as pentane, hexane, and heptane; and an ether-based compound having 4 or less carbon atoms such as ethyl methyl ether and diethyl ether, and one of these can be used alone, or two or more thereof may be used in combination. Among these, pentane is preferable from the viewpoint of suppressing shrinkage of the resultant porous cellulose particles during drying.

The amount of the dispersion medium used for the dispersion medium replacement is preferably 100 parts by mass or greater, more preferably 200 parts by mass or greater, and is preferably 2000 parts by mass or less, more preferably 1000 parts by mass or less, still more preferably 600 parts by mass or less, per 100 parts by mass of the cellulose particles after the cleaning process. The amount of the dispersion medium used for the dispersion medium replacement is preferably 100 parts by mass or greater and 2000 parts by mass or less, more preferably 200 parts by mass or greater and 1000 parts by mass or less, and still more preferably 200 parts by mass or greater and 600 parts by mass or less per 100 parts by mass of the cellulose particles after the cleaning process.

The dispersion medium replacement can be performed by, for example, adding the cellulose particles after the cleaning process to the dispersion medium and stirring the mixture with a known apparatus. The temperature during mixing of the cellulose particles and the dispersion medium after the cleaning process is preferably 0°C or higher, more preferably 5°C or higher, still more preferably 15°C or higher, and is preferably 50°C or lower, more preferably 40°C or lower, still more preferably 30°C or lower. The temperature during mixing of the cellulose particles and the dispersion medium after the cleaning process is preferably 0°C or higher and 50°C or lower, more preferably 5°C or higher and 40°C or lower, and still more preferably 15°C or higher and 30°C or lower.

The stirring speed during mixing of the cellulose particles and the dispersion medium after the cleaning process is appropriately set depending on production scale and temperature, but from the viewpoint of sufficiently dispersing the cellulose particles, the stirring speed is preferably 100 rpm or greater, more preferably 200 rpm or greater, and is preferably 2000 rpm or less, more preferably 1500 rpm or less, still more preferably 1000 rpm or less, even more preferably 800 rpm or less. The stirring speed during mixing of the cellulose particles and the dispersion medium after the cleaning process is preferably 100 rpm or greater and 2000 rpm or less, more preferably 200 rpm or greater and 1500 rpm or less, still more preferably 200 rpm or greater and 1000 rpm or less, and even more preferably 200 rpm or greater and 800 rpm or less.

The stirring time during mixing of the cellulose particles and the dispersion medium after the cleaning process is appropriately set depending on the production scale and temperature, and is usually 0.2 hours or more and 12 hours or less, and preferably 0.5 hours or more and 6 hours or less.

By performing the dispersion medium replacement, a suspension containing purified cellulose particles is produced. The suspension can be subjected to solid-liquid separation by the same method as described above to collect the purified cellulose wet particles.

<Step (V)>

In step (V), the purified cellulose wet particles produced in step (IV) are subjected to a drying process to produce dry porous cellulose particles.

As a method of drying the purified cellulose wet particles, a freeze-drying method is preferably used from the viewpoint of suppressing shrinkage of the particles during drying and maintaining a porous structure. In addition, when the dispersion medium replacement is performed in step (IV), a drying process can be performed by reduced-pressure drying, drying with supercritical carbon dioxide, or the like.

The freeze-drying is preferably performed in which the purified cellulose wet particles are subjected to preliminary freezing and then subjected to primary drying and secondary drying.

The preliminary freezing is preferably conducted by rapid freezing at a temperature of -200°C or higher and -50°C or lower under normal pressure. It is preferable that the primary drying is conducted in which ice in the preliminarily frozen product undergoes sublimation preferably under a vacuum of 0.1 Pa or higher and 100 Pa or lower and at a temperature of -20°C or higher and -5°C or lower, and then the temperature is raised to 20°C or higher and 40°C or lower under a vacuum of 0.1 Pa or higher and 100 Pa or lower to conduct the secondary drying.

[Cosmetics]

The present invention further provides a cosmetic containing the porous cellulose particles.

The cosmetic of the present invention contains the porous cellulose particles and thereby enables to impart an excellent sense of touch. Furthermore, since the porous cellulose particles are excellent in the inclusion of functional substances and tendency of the particles to deform, the cosmetic containing the porous cellulose particles can achieve a sustained-release of a functional substance to an object through application action.

From the viewpoint of effectively achieving the above-mentioned effects, the cosmetic of the present invention is preferably a skin cosmetic, and examples of the skin cosmetic include foundations, makeup bases, sunscreens, skin milk, and lotions.

The content of the porous cellulose particles in the cosmetic needs to be an amount capable of achieving the desired performance, and can be appropriately selected depending on the type, form, and the like of the cosmetic, and is usually in the range of 0.01 mass% or greater and 80 mass% or less in the cosmetic.

<Usage>

The porous cellulose particles of the present invention can be blended or used in usages other than cosmetics, for example, toiletry products, oral care products, quasi-drugs, medications, household products, agricultural products, and the like.

The porous cellulose particles of the present invention are made of cellulose derived from natural plants, and hence are environmentally friendly and can be suitably used as a substitute material for microplastics.

In addition to the embodiments described above, the present invention discloses the following.

<1> Porous cellulose particles having:

a compression modulus of 50 MPa or less,
a specific surface area of 100 $m^2/g$ or greater and less than 500 $m^2/g$, and
a pore volume of 1.5 mL/g or greater.

<2> The porous cellulose particles according to <1>, wherein the compression modulus of the porous cellulose particles is preferably 40 MPa or less, more preferably 30 MPa or less, still more preferably 20 MPa or less, even more preferably 10 MPa or less, even more preferably 7.0 MPa or less, even more preferably 6.0 MPa or less, even more preferably 5.0 MPa or less, even more preferably 5.3 MPa or less, and even more preferably 5.2 MPa or less.

<3> The porous cellulose particles according to <1> or <2>, wherein the compression modulus of the porous cellulose particles is preferably 1.0 MPa or greater, more preferably 2.0 MPa or greater, even more preferably 3.0 MPa or greater, even more preferably 4.0 MPa or greater, and even more preferably 4.9 MPa or greater.

<4> The porous cellulose particles according to any one of <1> to <3>, wherein the median size of the porous cellulose particle is 75 $\mu$m or less, as measured by a dry method.

<5> The porous cellulose particles according to any one of <1> to <4>, wherein the median size ($D_{50}$) of the porous cellulose particle is preferably 75 $\mu$m or less, more preferably 70 $\mu$m or less, still more preferably 65 $\mu$m or less, even more preferably 55 $\mu$m or less, even more preferably 40 $\mu$m or less, and even more preferably 30 $\mu$m or less, as measured by a dry method.

<6> The porous cellulose particles according to any one of <1> to <5>, wherein the median size ($D_{50}$) of the porous cellulose particle is preferably 5 $\mu$m or greater, more preferably 10 $\mu$m or greater, and still more preferably 15 $\mu$m or greater, as measured by a dry method.

<7> The porous cellulose particles according to any one of <1> to <6>, wherein the specific surface area of the porous cellulose particle is preferably 110 $m^2/g$ or greater, more preferably 120 $m^2/g$ or greater, still more preferably 130 $m^2/g$ or greater, even more preferably 135 $m^2/g$ or greater, even more preferably 140 $m^2/g$ or greater, and even more preferably 144 $m^2/g$ or greater.

<8> The porous cellulose particles according to any one of <1> to <7>, wherein the specific surface area of the porous cellulose particle is preferably 200 $m^2/g$ or less, more preferably 180 $m^2/g$ or less, and still more preferably 150 $m^2/g$ or less.

<9> The porous cellulose particles according to any one of <1> to <8>, wherein the pore volume of the porous cellulose particle is preferably 2.0 mL/g or greater, more preferably 2.5 mL/g or greater, still more preferably 3.0 mL/g or greater, even more preferably 3.5 mL/g or greater, and even more preferably 4.0 mL/g or greater.

<10> The porous cellulose particles according to any one of <1> to <9>, wherein the pore volume of the porous cellulose particle is preferably 8.0 mL/g or less, more preferably 7.0 mL/g or less, still more preferably 6.0 mL/g or less, and even more preferably 5.0 mL/g or less.

<11> The porous cellulose particles according to any one of <1> to <10>, wherein the sphericity of the porous cellulose particle is preferably 60% or greater, more preferably 70% or greater, and still more preferably 80% or greater.

<12> The porous cellulose particles according to any one of <1> to <11>, wherein the surface pore size of the porous cellulose particle is preferably 50 nm or greater, more preferably 100 nm or greater, and still more preferably 200 nm or greater.

<13> The porous cellulose particles according to any one of <1> to <12>, wherein the surface pore size of the porous cellulose particle is preferably 800 nm or less, more preferably 600 nm or less, and still more preferably 500 nm or less.

<14> The porous cellulose particles according to any one of <1> to <13>, wherein the porous cellulose particles are porous cellulose particles without chemical modification.

<15> The porous cellulose particles according to any one of <1> to <14>, wherein the porous cellulose particles are non-crosslinked particles.

<16> The porous cellulose particles according to any one of <1> to <15>, wherein cellulose that constitutes the porous cellulose particles is made of crystalline cellulose of cellulose type II or amorphous cellulose.

<17> The porous cellulose particles according to any one of <1> to <16>, wherein the content of cellulose in the porous cellulose particle is preferably 95 mass% or greater, more preferably 99 mass% or greater, and still more preferably substantially 100 mass%.

<18> A cosmetic including the porous cellulose particles described in any one of <1> to <17>.

<19> A method of producing the porous cellulose particles described in any one of <1> to <17>, including the following steps (I) to step (V) in this order:

step (I): mixing raw material cellulose and an alkaline aqueous solution to prepare a cellulose aqueous solution;
step (II): mixing the cellulose aqueous solution with an organic solvent to prepare a cellulose emulsion;
step (III): mixing the cellulose emulsion and a non-cellulose-dissolving solvent to precipitate crude cellulose particles, thereby producing a suspension containing the crude cellulose particles;

step (IV): subjecting the suspension containing the crude cellulose particles to solid-liquid separation, and then cleaning the resultant crude cellulose wet particles to produce purified cellulose wet particles; and

step (V): subjecting the purified cellulose wet particles to a drying process.

<20> The method of producing the porous cellulose particles according to <19>, wherein the raw material cellulose used in step (I) is preferably a chemically pure cellulose without chemical modification, and more preferably wood or pulp.

<21> The method of producing the porous cellulose particles according to <19> or <20>, wherein a shape of the raw material cellulose used in step (I) is in powder form, sheet form, and flocculent form, and preferably is in powder form.

<22> The method of producing the porous cellulose particles according to any one of <19> to <21>, wherein a degree of polymerization of the raw material cellulose used in step (I) is preferably 10 or greater, more preferably 50 or greater, still more preferably 100 or greater, even more preferably 150 or greater, and preferably 1000 or less, more preferably 500 or less, still more preferably 300 or less.

<23> The method of the producing porous cellulose particles according to any one of <19> to <22>, wherein the raw material cellulose used in step (I) is preferably crystalline cellulose, and more preferably crystalline cellulose of cellulose type I.

<24> The method of the producing porous cellulose particles according to any one of <19> to <23>, wherein the raw material cellulose used in step (I) is in a powder form, and the median size of the raw material cellulose is preferably 10 $\mu$m or greater, more preferably 20 $\mu$m or greater, and is preferably 500 $\mu$m or less, more preferably 300 $\mu$m or less, still more preferably 200 $\mu$m or less, even more preferably 150 $\mu$m or less.

<25> The method of producing the porous cellulose particles according to any one of <19> to <24>, wherein an alkaline compound used in the alkaline aqueous solution in step (I) is preferably an alkaline metal hydroxide, more preferably one or more selected from the group consisting of sodium hydroxide and potassium hydroxide, and still more preferably sodium hydroxide.

<26> The method of producing the porous cellulose particles according to any one of <19> to <25>, wherein a concentration of the alkaline compound in the alkaline aqueous solution used in step (I) is preferably 1 mass% or greater, more preferably 2 mass% or greater, and still more preferably 3 mass% or greater, and is preferably 40 mass% or less, more preferably 30 mass% or less, and still more preferably 25 mass% or less.

<27> The method of producing the porous cellulose particles according to any one of <19> to <26>, wherein in step (I), alkaline aqueous solutions having different concentrations are mixed with raw material cellulose multiple times.

<28> The method of producing the porous cellulose particles according to any one of <19> to <27>, wherein in step (I), raw material cellulose is mixed with an alkaline aqueous solution A containing an alkaline compound at a concentration of 1 mass% or greater and 10 mass% or less, and then an alkaline aqueous solution B containing an alkaline compound at a concentration of more than 10 mass% and 40 mass% or less is added thereto and mixed to prepare a cellulose aqueous solution.

<29> The method of producing the porous cellulose particles according to <28>, wherein the concentration of the alkaline compound in the alkaline aqueous solution A is more preferably 2 mass% or greater and 8 mass% or less, and still more preferably 2 mass% or greater and 5 mass% or less.

<30> The method of producing the porous cellulose particles according to <28> or <29>, wherein the concentration of the alkaline compound in the alkaline aqueous solution B is more preferably 15 mass% or greater and 30 mass% or less, and still more preferably 20 mass% or greater and 25 mass% or less.

<31> The method of producing the porous cellulose particles according to any one of <28> to <30>, wherein a mass ratio (A/B) of the alkaline aqueous solution A to the alkaline aqueous solution B is preferably 1 or greater and 10 or less, more preferably 2 or greater and 8 or less, and still more preferably 3 or greater and 6 or less.

<32> The method of producing the porous cellulose particles according to any one of <19> to <31>, wherein in step (I), a temperature during mixing of the raw material cellulose and the alkaline aqueous solution is preferably 10°C or lower, more preferably 5°C or lower, and still more preferably 0°C or lower, and is preferably -20°C or higher, more preferably -10°C or higher, and still more preferably -5°C or higher.

<33> The method of producing the porous cellulose particles according to any one of <28> to <30>, wherein in step (I), raw material cellulose is added to the alkaline aqueous solution A and stirred and mixed, then a temperature of the mixture is adjusted to preferably 10°C or lower, more preferably 5°C or lower, and still more preferably 0°C or lower, and also preferably -20°C or higher, more preferably -10°C or higher, and still more preferably -5°C or higher, and then the alkaline aqueous solution B is added thereto and mixed.

<34> The method of producing the porous cellulose particles according to any one of <19> to <33>, wherein the cellulose aqueous solution produced in step (I) contains cellulose at a concentration of 1% by mass or greater and 8% by mass or less.

<35> The method of producing the porous cellulose particles according to any one of <19> to <34>, wherein the concentration of the alkaline compound in the cellulose aqueous solution obtained in step (I) is preferably 0.5 mass% or greater, more preferably of 1 mass% or greater, still more preferably of 2 mass% or greater, even more preferably of 3 mass% or greater, and even more preferably of 5 mass% or greater, and preferably of 15 mass% or less, more preferably of 12 mass% or less, and still more preferably of 10 mass% or less.

<36> The method of producing the porous cellulose particles according to any one of <19> to <35>, wherein the organic solvent used in step (II) is a hydrocarbon solvent, more preferably a chain aliphatic hydrocarbon, still more preferably one or more selected from the group consisting of n-pentane, n-hexane, n-heptane, n-octane, isooctane, decane, isodecane, dodecane, isododecane, tetradecane, hexadecane, and octadecane, and even more preferably one or more selected from the group consisting of n-octane, isooctane, n-decane, isodecane, n-dodecane, and isododecane.

<37> The method of producing the porous cellulose particles according to any one of <19> to <36>, wherein an emulsifier is further mixed in step (II).

<38> The method of producing the porous cellulose particles according to <37>, wherein the emulsifier is a nonionic surfactant, preferably one or more selected from the group consisting of a sorbitan fatty acid ester, a polyoxyethylene alkyl ether, a sucrose fatty acid ester, and a polyether-modified silicone, more preferably a sucrose fatty acid ester, still more preferably one or more selected from the group consisting of sucrose palmitate, sucrose oleate, sucrose stearate, sucrose erucate, and sucrose behenate, and even more preferably one or more selected from the group consisting of sucrose erucate and sucrose behenate.

<39> The method of producing the porous cellulose particles according to <38>, wherein an HLB of the nonionic surfactant used as the emulsifier is preferably 1 or greater and 10 or less, more preferably 1 or greater and 8 or less, still more preferably 1 or greater and 6 or less, even more preferably 1 or greater and 5 or less, even more preferably 1 or greater and 4 or less, and even more preferably 1 or greater and 3 or less.

<40> The method of producing the porous cellulose particles according to any one of <37> to <39>, wherein a mixing amount of an emulsifier mixed in step (II) is preferably 0.1 parts by mass or greater, more preferably 0.5 parts by mass or greater, and still more preferably 1.0 parts by mass or greater, and is preferably 20 parts by mass or less, more preferably 10 parts by mass or less, and still more preferably 5.0 parts by mass or less, per 100 parts by mass of the organic solvent.

<41> The method of producing the porous cellulose particles according to any one of <19> to <40>, wherein in step (II), a temperature during mixing of the cellulose aqueous solution and the organic solvent is preferably 20°C or lower, more preferably 10°C or lower, and still more preferably 5°C or lower, and is preferably -20°C or higher, more preferably -10°C or higher, and still more preferably -5°C or higher.

<42> The method of producing the porous cellulose particles according to any one of <19> to <41>, wherein in step (II), a stirring speed during mixing of the cellulose aqueous solution and the organic solvent is preferably 1000 rpm or greater, more preferably 3000 rpm or greater, still more preferably 5000 rpm or greater, and even more preferably 5500 rpm or greater, and is preferably 15000 rpm or less, more preferably 12000 rpm or less, still more preferably 10000 rpm or less, and even more preferably 8500 rpm or less.

<43> The method of producing the porous cellulose particles according to any one of <19> to <42>, wherein the non-cellulose-dissolving solvent used in step (III) is an alcohol having 4 or less carbon atoms.

<44> The method of producing the porous cellulose particles according to any one of <19> to <43>, wherein the non-cellulose-dissolving solvent used in step (III) is preferably one or more selected from the group consisting of ethanol, 2-propanol, 1-butanol, 2-butanol, and 2-methyl-1-propanol, and more preferably ethanol.

<45> The method of producing the porous cellulose particles according to any one of <19> to <44>, wherein a mixing amount of the non-cellulose-dissolving solvent used in step (III) is preferably 50 parts by mass or greater, more preferably 100 parts by mass or greater, and still more preferably 200 parts by mass or greater, and is preferably 1000 parts by mass or less, more preferably 500 parts by mass or less, and still more preferably 400 parts by mass or less, per 100 parts by mass of the cellulose emulsion.

<46> The method of producing the porous cellulose particles according to any one of <19> to <45>, wherein an acid is further mixed in step (III).

<47> The method of producing the porous cellulose particles according to <46>, wherein the acid mixed in step (III) is preferably an organic acid, and more preferably a carboxylic acid having 4 or less carbon atoms.

<48> The method of producing the porous cellulose particles according to <46> or <47>, wherein a mixing amount of the acid mixed in step (III) is preferably 1.0 equivalent or greater, more preferably 1.2 equivalent or greater, still more preferably 1.4 equivalent or greater, and is preferably 3.0 equivalent or less, more preferably 2.0 equivalent or less, still more preferably 1.8 equivalent or less with respect to the alkaline compound used in step (I).

<49> The method of producing the porous cellulose particles according to any one of <46> to <48>, wherein in step (III), the cellulose emulsion and the non-cellulose-dissolving solvent are mixed, and then an acid is mixed.

<50> The method of producing the porous cellulose particles according to any one of <19> to <49>, wherein in step (III), a temperature during mixing of the cellulose emulsion and the non-cellulose-dissolving solvent is preferably 0°C or higher, more preferably 5°C or higher, and still more preferably 15°C or higher, and is preferably 50°C or lower, more preferably 40°C or lower, and still more preferably 30°C or lower.

<51> The method of producing the porous cellulose particles according to any one of <19> to <50>, wherein in step (III), a stirring speed during mixing of the cellulose emulsion and the non-cellulose-dissolving solvent is preferably 100 rpm or greater, more preferably 200 rpm or greater, and is preferably 2000 rpm or less, more preferably 1500 rpm or less, still more preferably 1000 rpm or less, even more preferably 800 rpm or less.

<52> The method of producing the porous cellulose particles according to any one of <19> to <51>, wherein in step (III), a stirring time during mixing of the cellulose emulsion and the non-cellulose-dissolving solvent is 0.2 hours or greater and 12 hours or less, and preferably 0.5 hours or greater and 6 hours or less.

<53> The method of producing the porous cellulose particles according to any one of <19> to <52>, wherein in step (IV), the solid-liquid separation of the suspension containing the crude cellulose particles produced in step (III) is performed by centrifugation, filtration, decantation, or a combination thereof.

<54> The method of producing the porous cellulose particles according to any one of <19> to <53>, wherein in step (IV), the cleaning process of the crude cellulose wet particles obtained after the solid-liquid separation is performed with water, an organic solvent, or a combination thereof.

<55> The method of producing the porous cellulose particles according to <54>, wherein the organic solvent is a ketone-based solvent having 6 or less carbon atoms or an alcohol-based solvent having 6 or less carbon atoms, and is preferably acetone, methyl isobutyl ketone, ethanol, or 2-propanol.

<56> The method of producing the porous cellulose particles according to any one of <19> to <55>, wherein in step (IV), the cellulose particles obtained after the cleaning process are dispersed in a dispersion medium to perform dispersion medium replacement, and reduced-pressure drying is further performed.

<57> The method of producing the porous cellulose particles according to <56>, wherein in step (IV), the dispersion medium used for the dispersion medium replacement is an organic solvent whose surface tension is preferably 20 mN/m or less at 25°C, more preferably 18 mN/m or less at 25°C, and is preferably pentane.

<58> The method of producing the porous cellulose particles according to <56> or <57>, wherein in step (IV), an amount of the dispersion medium used for the dispersion medium replacement is preferably 100 parts by mass or greater, more preferably 200 parts by mass or greater, and preferably 2000 parts by mass or less, more preferably 1000 parts by mass or less, still more preferably 600 parts by mass or less, per 100 parts by mass of the cellulose particles after the cleaning process.

<59> The method of producing the porous cellulose particles according to any one of <56> to <58>, wherein in step (IV), a temperature during mixing of the cellulose particles after the cleaning process and the dispersion medium is preferably 0°C or higher, more preferably 5°C or higher, and still more preferably 15°C or higher, and is preferably 50°C or lower, more preferably 40°C or lower, and still more preferably 30°C or lower.

<60> The method of producing the porous cellulose particles according to any one of <56> to <59>, wherein in step (IV), a stirring speed during mixing of the cellulose particles after the cleaning process and the dispersion medium is preferably 100 rpm or greater, more preferably 200 rpm or greater, and is preferably 2000 rpm or less, more preferably 1500 rpm or less, still more preferably 1000 rpm or less, even more preferably 800 rpm or less.

<61> The method of producing the porous cellulose particles according to any one of <56> to <60>, wherein in step (IV), the suspension obtained by the dispersion medium replacement is subjected to solid-liquid separation to collect purified cellulose wet particles.

<62> The method of producing the porous cellulose particles according to any one of <19> to <61>, wherein in step (V), the method of the drying process is a freeze drying method.

<63> The method of producing the porous cellulose particles according to <62>, wherein the freeze drying method is a method in which the purified cellulose wet particles are subjected to preliminary freezing, and then subjected to primary drying and secondary drying.

<64> The method of producing the porous cellulose particles according to <63>, wherein in the preliminary freezing, rapid freezing is conducted at a temperature of 200°C or higher and -50°C or lower under normal pressure, primary drying is conducted in which ice contained in the preliminarily frozen product undergoes sublimation preferably under a vacuum of 0.1 Pa or higher and 100 Pa or lower and at a temperature of -20°C or higher and -5°C or lower, and then the temperature is raised to 20°C or higher and 40°C or lower under a vacuum of 0.1 Pa or higher and 100 Pa or lower to conduct secondary drying.

Examples

Hereinafter, the present invention will be specifically described with reference to Examples, but the present invention is not limited to these Examples.

Various measurement and evaluation methods are as follows.

<X-ray diffraction intensity>

The X-ray diffraction intensity is measured with an X-ray diffraction apparatus ("MiniFlex-II" available from Rigaku Corporation) under the following conditions.

The measurement conditions are as follows.

X-ray source: Cu/K$\alpha$-radiation
Measurement range: $2\theta$ = 5-50°

The measurement sample was prepared by compression into a pellet having an area of 320 mm$^2$ × a thickness of 1 mm. The measurement is performed at X-ray scan speed of 5°/min.

The cellulose type II crystal has a peak ($I_{20.0}$) corresponding to the 110 plane appearing at a diffraction angle $2\theta$ = 20.0°. The cellulose type II crystallinity ($X$ [%]) is determined by the following equation using the peak intensity ($I_{15.0}$) at $2\theta$ = 15.0° representing the amorphous portion.

$$X = [(I_{20.0} - I_{15.0})/I_{20.0}] \times 100$$

<Compression modulus>

The compression modulus of the cellulose particles is measured with a micro compression tester ("MCT-510" available from Shimadzu Corporation). Specifically, the cellulose particles are placed on a measurement stage attached to the apparatus, and the size d is measured. An indenter (Ø 50 $\mu$m) is lowered at a constant loading rate (mN/sec) to compress the particles until a specified test force (0.98 mN) is attained. The compressive stress is calculated by the particle size d ($\mu$m) and the test force P (mN) by using the following equation.

$$\text{Compressive stress (MPa)} = 2.48 \times P \text{ (mN)}/(\pi \times (d \text{ (}\mu\text{m)})^2)$$

The compressive strain is calculated by the displacement x ($\mu$m) and the particle size d ($\mu$m) by using the following equation.

$$\text{Compressive strain (\%)} = x \text{ (}\mu\text{m)}/d \text{ (}\mu\text{m)} \times 100$$

A stress-strain curve is created from the calculated compressive stress and compressive strain, and the compression modulus is calculated from the gradient in the elastic region (0-10%). The measurements are performed seven times, and the average value of five measurements excluding the maximum value and the minimum value is used as the test result.

<Median size>

The median size ($D_{50}$) of the cellulose particles is measured with a laser diffraction particle size analyzer ("LS 13 320" available from Beckman Coulter, Inc.). Specifically, 50 mg of dried cellulose particles are weighed in a measurement cell, and measurement is taken with the use of a tornado dry powder module to determine a particle size corresponding to 50% in the volume distribution of the particle size. In the measurement, the measured value 1.469 is entered as the refractive index of cellulose.

<Specific surface area>

The specific surface area of the cellulose particles is measured by the following method. Based on mercury intrusion porosimetry, a mercury porosimeter ("Auto Pore IV 9500" available from Shimadzu Corporation) is used to force mercury into the cellulose particles, and the total value $X$ (m$^2$) that is the sum of the surface area of the fine surface inside the cellulose particles and the surface area of the particle surface is determined. Specifically, about 0.05 g of cellulose particles are placed in a cell of the mercury porosimeter, measurement is carried out based on mercury intrusion porosimetry at a measurement pressure in a range of from 0.01 MPa to 210 MPa, and the value of X (m$^2$) is determined. The mass of the cellulose particles used as the measurement sample is denoted by $Y$ (g), and the value of $X$ (m$^2$)/Y (g) is determined as the specific surface area of the cellulose particles.

<Pore volume>

The pore volume of the cellulose particles is measured by the following method. Based on mercury intrusion porosimetry, a mercury porosimeter ("Auto Pore IV 9500" available from Shimadzu Corporation) is used to force mercury into the cellulose particles and the total volume $Z$ (mL) of mercury that has entered into the pores in the cellulose particles and the gaps between the cellulose particles is determined. Specifically, about 0.05 g of cellulose particles are placed in a cell of the mercury porosimeter, measurement is carried out based on mercury intrusion porosimetry at a measurement pressure in a range of from 0.01 MPa to 210 MPa, and the value of $Z$ (mL) is determined. The mass of the cellulose particles used as the measurement sample is denoted by $Y(g)$, and the value of $Z$ (mL)/$Y$ (g) is determined as the pore volume of the cellulose particles.

<Surface pore size>

The surface pore size of the cellulose particles is measured by the following method. Based on mercury intrusion porosimetry, a mercury porosimeter ("Auto Pore IV 9500" available from Shimadzu Corporation) is used to force mercury into the cellulose particles, and the pore distribution in the cellulose particles is determined. Specifically, first, about 0.05 g of cellulose particles are placed in a cell of the mercury porosimeter, measurement is carried out based on mercury intrusion porosimetry at a measurement pressure in a range of from 0.01 MPa to 210 MPa, and the pore volume of the cellulose particles: $Z$ (mL)/$Y$ (g) is determined by the same method as described above. A cumulative pore distribution curve is obtained by plotting the pore size (nm) on the horizontal axis and the pore volume (mL/g) on the vertical axis. Then, a derivative of the (cumulative) pore volume with respect to the pore size, that is, an increment of the pore volume at each pore size is determined as a differential pore volume (mL/g). A pore distribution curve is obtained by plotting the pore size (nm) on the horizontal axis and the differential pore volume (mL/g) on the vertical axis. In the resulting pore distribution curve, the mode of the pore sizes in a region of the pore size of 1000 nm or below is determined as the surface pore size of the particles.

<Sphericity>

The sphericity of the cellulose particles is measured with a dynamic image analyzer (CAMSIZER X2 available from MICROTRAC MRB). Specifically, 20 mg of cellulose particles after drying is placed into the apparatus feeder, and the particles are dispersed at an air dispersion pressure of 30 kPa. The dispersion is placed in the dynamic image analyzer to obtain an image. From the image, the sphericity (%) of one particle is calculated by the following formula. The average value of approximately 5000 images obtained is defined as the sphericity.

$$\text{Sphericity (\%)} = 4\pi \times \text{area of particle (m}^2)/(\text{outer periphery of particle (m)})^2 \times 100$$

<Tactile sensation>

About 5 mg of cellulose particles were applied in an area of 4 cm $\times$ 5 cm on the forearm of the right arm of a specialized panelist at 0.25 mg/cm$^2$, and softness and an absence of grittiness during application were evaluated by sensory evaluation according to the following criteria.
5: very soft and smooth, 4: soft and smooth, 3: no hardness and no grittiness, 2: hard and gritty, 1: very hard and gritty.

<Inclusion ratio of functional substance (Nile Red)>

About 50 mg of cellulose particles is weighed, and mixed with a solution in which about 50 mg of Nile Red (available from Tokyo Chemical Industry Co., Ltd.) is dissolved as a functional substance in 20 mL of ethanol. After stirring this solution overnight, ethanol is removed by vacuum drying at 60°C to obtain composite particles including cellulose particles and Nile Red. The obtained composite particles are taken out, and Nile Red contained in the particles is extracted with 50 mL o-xylene. The amount of Nile Red extracted is determined by ultraviolet-visible absorption spectroscopy, and the inclusion ratio is calculated by the following equation.

Inclusion ratio (%) = (amount of Nile Red extracted (mg))/(amount of Nile Red used (mg)) $\times$ 100

The inclusion ratio is scored according to the following criteria.
5: 80% or greater, 4: 60% or greater and less than 80%, 3: 40% or greater and less than 60%, 2: 20% or greater and less than 40%, 1: less than 20%

<Deformation due to abrasion>

About 20 mg of cellulose particles is weighed and placed on an artificial leather (Laforet S2923, 5 cm × 4 cm). A surface property tester ("Tribo Gear TYPE14 " available from Shinto Scientific Co., Ltd.) is used to perform 20 reciprocal abrasive actions at a moving distance of 50 mm and a moving speed of 2000 mm/min under a vertical load of 200 g equivalent to a coating operation. The cellulose particles remaining on the artificial leather surfaces are observed with a scanning electron microscope (SEM, "JSM-IT- 500HR" available from JEOL Ltd.) under the conditions of an accelerating voltage of 5.0 kV and an observation magnification of 500 times. Among the 20 cellulose particles in the observation image, the percentage of particles flattened (deformed) due to abrasion is determined as a deformation ratio, and scored according to the following criteria.
5: 80% or greater, 4: 60% or greater and less than 80%, 3: 40% or greater and less than 60%, 2: 20% or greater and less than 40%, 1: less than 20%

Example 1 (Production and evaluation of porous cellulose particles)

(Step (I))

As the raw material cellulose, crystalline cellulose powder of cellulose type I ("Avicel PH-101" available from Asahi Kasei Corporation, degree of polymerization: 170, median size: 50 $\mu$m, water content: 6%) was used.
Into 189.4 g of a dilute aqueous solution of NaOH (NaOH concentration: 4.2 mass%), 10.6 g of the cellulose powder was added, and the mixture was cooled to -2°C. Thereafter, while the mixture being maintained at a temperature of -2°C, 50 g of a concentrated aqueous solution of NaOH (NaOH concentration: 22 mass%) was added thereto and stirred for 1 hour to dissolve the raw material cellulose and thereby obtaining a cellulose aqueous solution.
The cellulose concentration and NaOH concentration in the resulting cellulose aqueous solution were found to be 4 mass% and 7.6 mass%, respectively.

(Step (II))

Into the cellulose aqueous solution, 350 g of isododecane and 3.5 g of sucrose erucate ("RYOTO Sugar Ester ER-290" available from Mitsubishi Chemical Corporation, HLB: 2, monoester content: about 2%) were added as an emulsifier. A homo-mixer ("MARK II 2.5 type" available from PRIMIX Corporation) was used to stir the mixture at 5°C and 7000 rpm for 5 minutes and thereby emulsifying the mixture to yield a water-in-oil type emulsion of cellulose. The emulsion droplet size in the emulsion was measured with a laser diffraction/scattering particle size distribution analyzer ("LA-960 V2" available from HORIBA, Ltd.) and found to be within a range of from 10 to 80 $\mu$m.

(Step (III))

The whole amount of the emulsion obtained in step (II) was added to 1500 g of alcohol (ethanol) as a non-cellulose-dissolving solvent, and the mixture was stirred at 400 rpm for 1 hour at room temperature (25°C) with a stirring blade to precipitate crude cellulose particles. Then, 42.8 g (1.5 equivalent to NaOH) of acetic acid was added thereto for neutralization to obtain a suspension containing crude cellulose particles.

(Step (IV))

The suspension obtained in step (III) was subjected to filtration under reduced pressure (700 hPa) with filter paper ("OMNIPORE DISC PTFE PHILIC 1.0 $\mu$M 90MM WH PLN 25/PK" available from Millipore, mesh size of 1 $\mu$m) to perform solid-liquid separation. Acetone (300 parts by mass per 100 parts by mass of cellulose wet particles) was added to the collected wet particles, the mixture was stirred at room temperature for 1 hour, and then solid-liquid separation was performed again. This operation was repeated twice. Subsequently, water (300 parts by mass per 100 parts by mass of the cellulose wet particles) was added thereto and the mixture was stirred at room temperature for 1 hour, and then solid-liquid separation was performed again. This operation was repeated twice. After the cleaning step, the obtained suspension was subjected to solid-liquid separation again to collect wet particles of purified cellulose.

(Step (V))

The wet particles of purified cellulose collected in step (IV) were rapidly frozen in a dry ice-ethyl alcohol bath at -72°C and then freeze-dried in a vacuum of 100 Pa or less to yield dry particles of purified porous cellulose.

The obtained porous cellulose particles were evaluated by the above-described method. The results are shown in Table 1.

Examples 2 to 3 and 5 to 6

Porous cellulose particles were produced in the same manner as in Example 1 except that the conditions of steps (I) to (V) were changed from Example 1 as shown in Table 1, and the porous cellulose particles were evaluated. The results are shown in Table 1.

Example 4

After steps (I) to (IV) were performed in the same manner as in Example 1, the following operation was further performed to produce porous cellulose particles, and evaluation thereof was performed. The results are shown in Table 1.

Pentane (300 parts by mass with respect to the cellulose wet particles) was added to the purified cellulose wet particles collected in step (IV), and the mixture was stirred at 400 rpm for 1 hour and at room temperature with a stirring blade, and then solid-liquid separation was performed again in the same manner as described above. This operation was repeated twice. After the above operation, the obtained wet particles of purified cellulose were dried overnight under reduced pressure at a vacuum of 50 kPa or less to obtain dry particles of purified porous cellulose.

Comparative Examples 1 to 3

Evaluation was performed using commercially available cellulose particles described in Table 1 as cellulose particles. The results are shown in Table 1.

[Table 1]

Table 1

| | | Examples | | | | | | Comparative Examples | | |
|---|---|---|---|---|---|---|---|---|---|---|
| | | 1 | 2 | 3 | 4 | 5 | 6 | 1 | 2 | 3 |
| Step (I) | Cellulose concentration (mass%) | 4 | 8 | 4 | 4 | 4 | 4 | "CELLUFLOW C25" available from JNC CORPORATION | "CELLULOBEADS USF" available from DAITO KASEI KOGYO CO., LTD. | "CELLULOBEADS D5" available from DAITO KASEI KO-GYO CO., LTD. |
| Step (II) | Rotation speed of homo-mixer (rpm) | 7000 | 12000 | 3000 | 10000 | 7000 | 2000 | | | |
| | Dispersant/dispersion medium | ER-290 Iso-dodecane | ER-290 Iso-dodecane | ER-290 Iso-dodecane | ER-290 Iso-dodecane | ER-290 Iso-dodecane | ER-290 Iso-dodecane | | | |
| | Emulsification time (min) | 5 | 7 | 3 | 6 | 5 | 3 | | | |
| | Emulsification tempera-ture (°C) | 5 | 5 | 5 | 5 | 5 | 5 | | | |
| Step (III) | Non-dissolving solvent | Ethanol | Ethanol | Ethanol | Ethanol | Ethanol | Ethanol | | | |
| | Amount of non-dissolving solvent (per 100 parts by mass of cellulose emulsion) | 300 | 300 | 300 | 300 | 300 | 300 | | | |
| Step (IV) | Cleaning solvent | (1) acetone (2) water | (1) acetone (2) water | (1) acetone (2) water | (1) acetone (2) water | (1) acetone (2) water | (1) acetone (2) water | | | |
| Step (V) | Drying method | Freeze-dry-ing | Freeze-dry-ing | Freeze-dry-ing | Reduced pressure | Freeze-dry-ing | Freeze-dry-ing | | | |
| | Solvent | - | - | - | Pentane | - | - | | | |

22

EP 4 620 997 A1

(continued)

| | | Examples | | | | | | Comparative Examples | | |
|---|---|---|---|---|---|---|---|---|---|---|
| | | 1 | 2 | 3 | 4 | 5 | 6 | 1 | 2 | 3 |
| Physical properties of particles | Compression modulus (MPa) | 5.0 | 48 | 5.1 | 2.8 | 4.8 | 5.4 | 48 | 30 | 180 |
| | Median size ($\mu$m) | 20 | 21 | 50 | 20 | 9 | 60 | 9 | 5 | 5 |
| | Specific surface area (m$^2$/g) | 145 | 140 | 139 | 102 | 132 | 140 | 72 | 110 | 10 |
| | Pore volume (mL/g) | 4.5 | 3.6 | 3.4 | 3.5 | 1.6 | 3.6 | 1.2 | 1.3 | 0.5 |
| | Surface pore size (nm) | 400 | 100 | 300 | 200 | 300 | 300 | 30 | < 10 | < 10 |
| | Sphericity (%) | 84 | 89 | 83 | 85 | 83 | 83 | 85 | 82 | 88 |
| Particle morphology | | Porous | Porous | Porous | Porous | Porous | Porous | Porous | Porous | Solid |
| Evaluation result | Tactile sensation | 5 | 3 | 4 | 5 | 5 | 3 | 2 | 3 | 2 |
| | Inclusion ratio of functional substance | 5 | 5 | 5 | 4 | 3 | 5 | 2 | 3 | 1 |
| | Deformation due to abrasive action | 5 | 3 | 5 | 5 | 4 | 5 | 1 | 1 | 1 |

EP 4 620 997 A1

It can be seen from Table 1 that the porous cellulose particles of the present Examples have an excellent tactile sensation when applied to the skin, and are also excellent in the inclusion of a functional substance and in tendency of the particles to deform during performing application action. **In** contrast, the cellulose particles of Comparative Examples were poor in any of the above-mentioned performances.

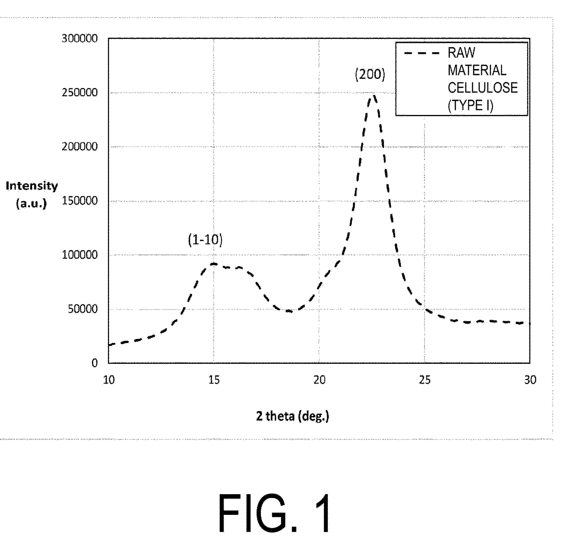

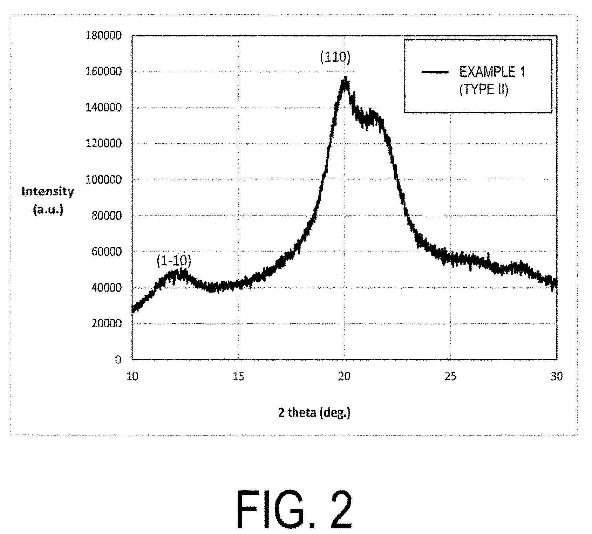

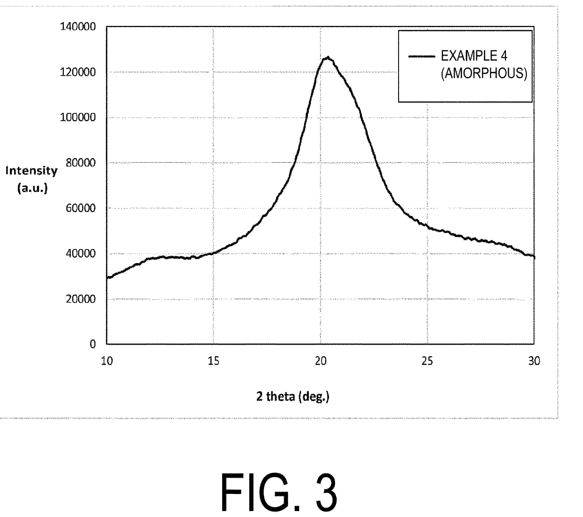

FIGs. 1 to 3 are X-ray diffraction profiles of the raw material cellulose (cellulose type I crystal) used in Examples, the porous cellulose particles (cellulose type II crystal) obtained in Example 1, and the porous cellulose particles (amorphous) obtained in Example 4, respectively. According to FIG. 2 in which a diffraction peak attributed to (11-0) plane appears at a diffraction angle $2\theta = 12.5°$, and a diffraction peak attributed to (110) plane appears at a diffraction angle $2\theta = 20.0°$, the porous cellulose particles obtained in Example 1 can be attributed to crystals of cellulose type II. FIG. 3 shows a broad peak, and hence the porous cellulose particles obtained in Example 4 can be attributed to amorphous cellulose.

Industrial Applicability

According to the present invention, it is possible to provide porous cellulose particles capable of imparting a soft feel when blended in a cosmetic, having less squeakiness during application, and also being excellent in the inclusion of a functional substance and tendency of particles to deform.

## Claims

1.  Porous cellulose particles having:

    a compression modulus of 50 MPa or less,
    a specific surface area of 100 m$^2$/g or greater and less than 500 m$^2$/g, and
    a pore volume of 1.5 mL/g or greater.

2.  The porous cellulose particles according to claim 1, wherein the porous cellulose particles have a median size of 75 μm or less as measured by a dry method.

3.  The porous cellulose particles according to claim 1 or 2, wherein the porous cellulose particles are porous cellulose particles without chemical modification.

4.  The porous cellulose particles according to any one of claims 1 to 3, wherein cellulose that constitutes the porous cellulose particles is made of crystalline cellulose of cellulose type II or amorphous cellulose.

5.  The porous cellulose particles according to any one of claims 1 to 4, wherein the porous cellulose particles contain cellulose in an amount of 95 mass% or greater.

6.  The porous cellulose particles according to any one of claims 1 to 5, wherein the porous cellulose particles have a surface pore size of 50 nm or greater and 800 nm or less.

7.  The porous cellulose particles according to any one of claims 1 to 6, wherein the porous cellulose particles have a sphericity of 60% or greater.

8.  A cosmetic comprising the porous cellulose particles described in any one of claims 1 to 7.

9.  A method of producing the porous cellulose particles described in any one of claims 1 to 7, the method comprising the following steps (I) to step (V) in this order:

    step (I): mixing raw material cellulose and an alkaline aqueous solution to prepare a cellulose aqueous solution;
    step (II): mixing the cellulose aqueous solution with an organic solvent to prepare a cellulose emulsion;
    step (III): mixing the cellulose emulsion and a non-cellulose-dissolving solvent to precipitate crude cellulose particles, thereby producing a suspension containing the crude cellulose particles;
    step (IV): subjecting the suspension containing the crude cellulose particles to solid-liquid separation, and then subjecting produced crude cellulose wet particles to a cleaning process, thereby producing purified cellulose wet particles; and
    step (V): subjecting the purified cellulose wet particles to a drying process, thereby producing porous cellulose particles.

10. The method of producing the porous cellulose particles according to claim 9, wherein the raw material cellulose used in step (I) is crystalline cellulose of cellulose type I.

11. The method of producing the porous cellulose particles according to claim 9 or 10, wherein the cellulose aqueous solution produced in step (I) contains cellulose at a concentration of 1 mass% or greater and 8 mass% or less.

12. The method of producing the porous cellulose particles according to any one of claims 9 to 11, wherein the organic solvent used in step (II) is a hydrocarbon solvent.

13. The method of producing the porous cellulose particles according to any one of claims 9 to 12, wherein an emulsifier is further mixed in step (II).

14. The method of producing the porous cellulose particles according to any one of claims 9 to 13, wherein the non-cellulose-dissolving solvent used in step (III) is an alcohol having 4 or less carbon atoms.

15. The method of producing the porous cellulose particles according to any one of claims 9 to 14, wherein an acid is further mixed in step (III).

# FIG. 1

# FIG. 2

FIG. 3

## INTERNATIONAL SEARCH REPORT

| International application No. |
| --- |
| **PCT/JP2023/041400** |

**A. CLASSIFICATION OF SUBJECT MATTER**

*C08J 3/16*(2006.01)i; *A61K 8/73*(2006.01)i; *A61Q 1/02*(2006.01)i; *A61Q 17/04*(2006.01)i; *A61Q 19/00*(2006.01)i; *C08B 15/08*(2006.01)i

FI:  C08J3/16 CEP; A61K8/73; A61Q1/02; A61Q19/00; A61Q17/04; C08B15/08

According to International Patent Classification (IPC) or to both national classification and IPC

**B. FIELDS SEARCHED**

Minimum documentation searched (classification system followed by classification symbols)

C08J3/16; A61K8/73; A61Q1/02; A61Q17/04; A61Q19/00; C08B15/08

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
| --- | --- | --- |
| A | WO 2022/118883 A1 (KAO CORPORATION) 09 June 2022 (2022-06-09)<br>entire text, all drawings | 1-15 |
| A | JP 2015-187255 A (FUJIFILM CORPORATION) 29 October 2015 (2015-10-29)<br>entire text, all drawings | 1-15 |
| A | WO 2020/004604 A1 (JGC CATALYSTS & CHEMICALS LTD.) 02 January 2020 (2020-01-02)<br>entire text, all drawings | 1-15 |
| A | WO 2019/189692 A1 (JGC CATALYSTS & CHEMICALS LTD.) 03 October 2019 (2019-10-03)<br>entire text, all drawings | 1-15 |
| A | JP 2019-178257 A (JGC CATALYSTS & CHEMICALS LTD.) 17 October 2019 (2019-10-17)<br>entire text, all drawings | 1-15 |

☑ Further documents are listed in the continuation of Box C.    ☑ See patent family annex.

| | |
| --- | --- |
| \* Special categories of cited documents:<br>"A" document defining the general state of the art which is not considered to be of particular relevance<br>"D" document cited by the applicant in the international application<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
| --- | --- |
| **06 February 2024** | **20 February 2024** |

| Name and mailing address of the ISA/JP | Authorized officer |
| --- | --- |
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (July 2022)

**INTERNATIONAL SEARCH REPORT**

| International application No. |
| --- |
| **PCT/JP2023/041400** |

### C. DOCUMENTS CONSIDERED TO BE RELEVANT

| Category* | Citation of document, with indication, where appropriate, of the relevant passages | Relevant to claim No. |
| --- | --- | --- |
| A | JP 2018-172578 A (JGC CATALYSTS & CHEMICALS LTD.) 08 November 2018 (2018-11-08)<br>entire text, all drawings | 1-15 |
| A | JP 61-241337 A (KANEBO LTD) 27 October 1986 (1986-10-27)<br>entire text, all drawings | 1-15 |
| A | JP 63-90501 A (KANEBO LTD) 21 April 1988 (1988-04-21)<br>entire text, all drawings | 1-15 |

Form PCT/ISA/210 (second sheet) (July 2022)

**INTERNATIONAL SEARCH REPORT**
Information on patent family members

International application No.

**PCT/JP2023/041400**

| Patent document cited in search report | | | Publication date (day/month/year) | Patent family member(s) | | | Publication date (day/month/year) |
|---|---|---|---|---|---|---|---|
| WO | 2022/118883 | A1 | 09 June 2022 | JP | 2022-88343 | A | |
| JP | 2015-187255 | A | 29 October 2015 | US | 2016/0355662 | A1 | |
| | | | | entire text, all drawings | | | |
| | | | | WO | 2015/137170 | A1 | |
| | | | | CN | 106062055 | A | |
| WO | 2020/004604 | A1 | 02 January 2020 | US | 2021/0259942 | A1 | |
| | | | | entire text, all drawings | | | |
| WO | 2019/189692 | A1 | 03 October 2019 | US | 2021/0000705 | A1 | |
| | | | | entire text, all drawings | | | |
| | | | | EP | 3778749 | A1 | |
| | | | | CN | 111801377 | A | |
| | | | | KR | 10-2020-0136888 | A | |
| JP | 2019-178257 | A | 17 October 2019 | (Family: none) | | | |
| JP | 2018-172578 | A | 08 November 2018 | US | 2018/0280256 | A1 | |
| | | | | entire text, all drawings | | | |
| | | | | CN | 108685765 | A | |
| | | | | KR | 10-2018-0111669 | A | |
| JP | 61-241337 | A | 27 October 1986 | US | 4902792 | A | |
| | | | | entire text, all drawings | | | |
| | | | | US | 5244734 | A | |
| | | | | EP | 200973 | A2 | |
| JP | 63-90501 | A | 21 April 1988 | (Family: none) | | | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP H0284401 A
- WO 2020004604 A
- WO 2015029790 A
- JP 2017014528 A
- JP 2021161246 A
- CN 101250267 A

**Non-patent literature cited in the description**

- *J. Soc. Cosm. Chem.*, vol. 5, 249-256